# EUROPEAN PATENT SPECIFICATION

(11) **EP 0 815 115 B1**
(45) Date of publication and mention of the grant of the patent: **28.05.2003**
(21) Application number: 95911700.3
(22) Date of filing: 14.02.1995
(51) Int. Cl.: C07H 21/00, A61K 38/18, C07K 14/50

(54) **KERATINOCYTE GROWTH FACTOR-2**
KERATINOZYTEN-WACHSTUMSFAKTOR 2
FACTEUR-2 DE CROISSANCE DES KERATINOCYTES

(43) Date of publication of application: 07.01.1998
(60) Divisional application: 03006708.6
(73) Proprietor: HUMAN GENOME SCIENCES, INC., Rockville, MD 20850-3338 (US)
(72) Inventor: GRUBER, Joachim, R., Gaithersburg, MD 20879 (US); DILLON, Patrick, J., Gaithersburg, MD 20879 (US)
(74) Representative: VOSSIUS & PARTNER
(86) International application number: US9501790
(87) International publication number: WO96025422

(56) References cited:
- EP-A- 0 619 370
- SCIENCE, Volume 245, issued 18 August 1989, P.W. FINCH et al., "Human KGF is FGF-Related With Properties of a Paracrine Effector of Epithelial Cell Growth", pages 752-755.
- PROC. NATL. ACAD. SCI. U.S.A., Volume 89, issued October 1992, M.J. KELLEY et al., "Emergence of the Keratinocyte Growth Factor Multigene Family During the Great Ape Radiation", pages 9287-9291.
- IN VITRO CELLULAR DEVELOPMENTAL BIOLOGY, Volume 27A, issued June 1991, G. YAN et al., "Sequence of Rat Keratinocyte Growth Factor (Heparin-Binding Growth Factor Type 7)", pages 437-438.
- MECHANISMS OF DEVELOPMENT, Volume 45, issued 1994, I.J. MASON et al., "FGF-7 (Keratinocyte Growth Factor) Expression During Mouse Development Suggests Roles in Myogenesis, Forebrain Regionalisation and Epithelial-Mesenchymal Interactions", pages 15-30.
- MOLECULAR AND CELLULAR BIOLOGY, Volume 13, Number 7, issued July 1993, M. MIYAMOTO et al., "Molecular Cloning of a Novel Cytokine cDNA Encoding the Ninth Member of the Fibroblast Growth Factor Family, Which Has a Unique Secretion Property", pages 4251-4259.

## Description

This invention relates to newly identified polynucleotides, polypeptides encoded by such polynucleotides, the use of such polynucleotides and polypeptides, as well as the production of such polynucleotides and polypeptides. More particularly, the polypeptide of the present invention is a Keratinocyte growth factor, sometimes hereinafter referred to as "KGF-2". The invention also relates to inhibiting the action of such polypeptides.

The fibroblast growth factor family has emerged as a large family of growth factors involved in soft-tissue growth and regeneration. It presently includes several members that share a varying degree of homology at the protein level, and that, with one exception, appear to have a similar broad mitogenic spectrum, i.e., they promote the proliferation of a variety of cells of mesodermal and neuroectodermal origin and/or promote angiogenesis.

The pattern of expression of the different members of the family is very different, ranging from extremely restricted expressions of some stages of development, to rather ubiquitous expression in a variety of tissues and organs. All the members appear to bind heparin and heparin sulfate proteoglycans and glycosaminoglycans and strongly concentrate in the extracellular matrix. KGF was originally identified as a member of the FGF family by sequence homology or factor purification and cloning.

Keratinocyte growth factor (KGF) was isolated as a mitogen for a cultured murine keratinocyte line (Rubin, J.S., et al., PNAS, USA, 86:802-806 (1989)). Unlike the other members of the FGF family, it has little activity on mesenchyme-derived cells but stimulates the growth of epithelial cells. The Keratinocyte growth factor gene encodes a 194-amino acid polypeptide (Finch, P.W., et al., Science, 245:752-755 (1989)). The N-terminal 64 amino acids are unique, but the remainder of the protein has about 30% homology to bFGF. KGF is the most divergent member of the FGF family. The molecule has a hydrophobic signal sequence and is efficiently secreted. Post-translational modifications include cleavage of the signal sequence and N-linked glycosylation at one site, resulting in a protein of 28 kDa. Keratinocyte growth factor is produced by fibroblast derived from skin and fetal lung, (Rubin, et al., (1989)). The Keratinocyte growth factor mRNA was found to be expressed in adult kidney, colon and ilium, but not in brain or lung (Finch, P.W., et al., Science, 245:752-755 (1989)). KGF displays the conserved regions within the FGF protein family. KGF binds to the FGF-2 receptor with high affinity.

The polypeptide of the present invention has been putatively identified as a member of the FGF family, more particularly the polypeptide has been putatively identified as KGF-2 as a result of amino acid sequence homology with other members of the FGF family.

In accordance with one aspect of the present invention, there are provided novel mature polypeptides which are KGF-2 as well as biologically active and diagnostically or therapeutically useful fragments, analogs and derivatives thereof. The polypeptides of the present invention are of human origin.

In accordance with one aspect of the present invention, there are provided nucleic acid molecules encoding human KGF-2, including mRNAs, DNAs, cDNAs, genomic DNA, as well as antisense analogs thereof and biologically active and diagnostically or therapeutically useful fragments thereof.

In accordance with another aspect of the present invention, there is provided a process for producing such polypeptide by recombinant techniques through the use of recombinant vectors, such as cloning and expression plasmids useful as reagents in the recombinant production of KGF-2 proteins, as well as recombinant prokaryotic and/or eukaryotic host cells comprising a human KGF-2 nucleic acid sequence.

The polypeptide, or a polynucleotide encoding such polypeptide can be used for therapeutic purposes, for example, to stimulate epithelial cell proliferation for the purpose of wound healing, and to stimulate hair follicle production and healing of dermal wounds.

In accordance with yet a further aspect of the present invention, there are provided antibodies against such polypeptides.

In accordance with another aspect of the present invention, there are provided nucleic acid probes comprising nucleic acid molecules of sufficient length to specifically hybridize to human KGF-2 sequences.

The polynucleotides and polypeptides of the present invention, can be used to identify antagonists to such polypeptides, which may be used to inhibit the action of such polypeptides, for example, to reduce scarring during the wound healing process and to prevent and/or treat tumor proliferation, diabetic retinopathy, rheumatoid arthritis and tumor growth.

They can also be used in diagnostic assays for detecting diseases or susceptibility to diseases related to mutations in KGF-2 nucleic acid sequences or over-expression of the polypeptides encoded by such sequences.

Such polypeptides, or polynucleotides encoding such polypeptides can also be used in a process for *in vitro* purposes related to scientific research, synthesis of DNA and manufacture of DNA vectors.

These and other aspects of the present invention should be apparent to those skilled in the art from the teachings herein.

The following drawings are illustrative of embodiments of the invention and are not meant to limit the scope of the invention as encompassed by the claims.

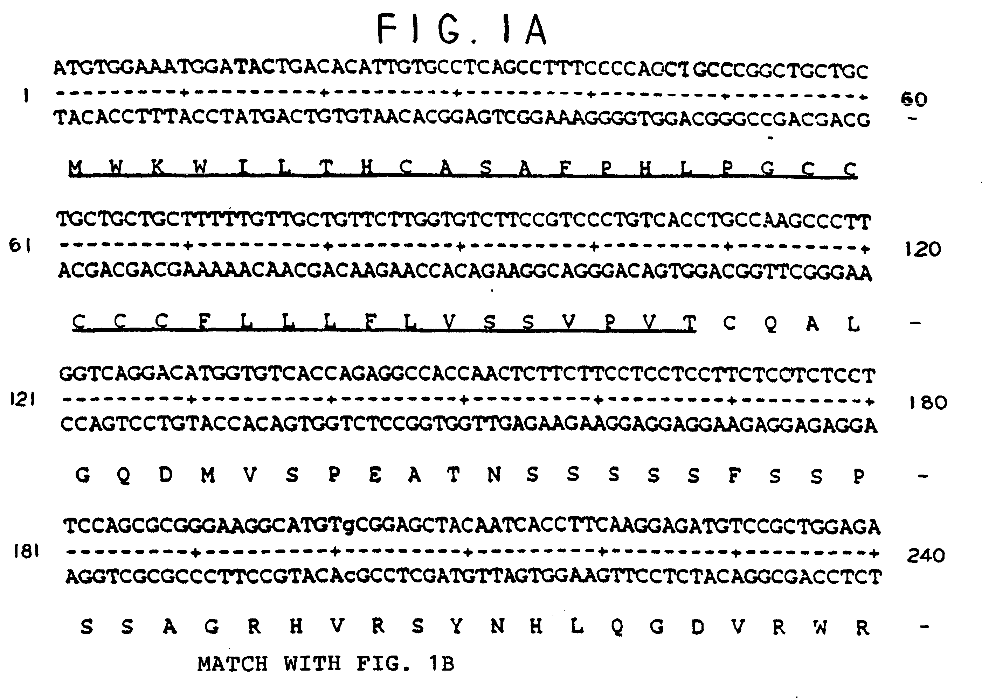

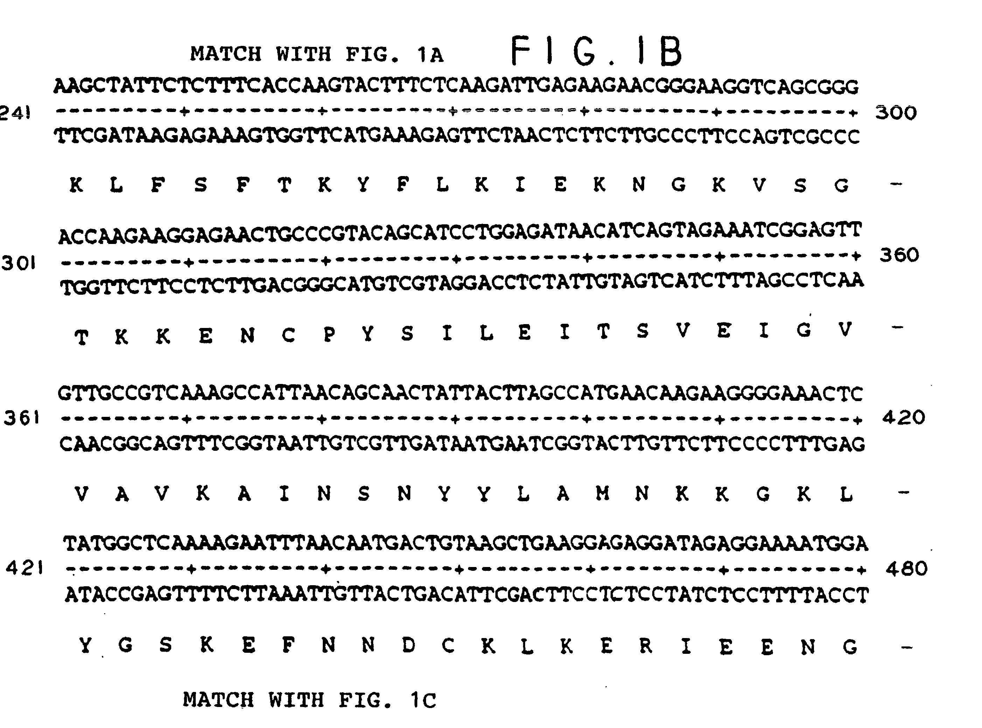

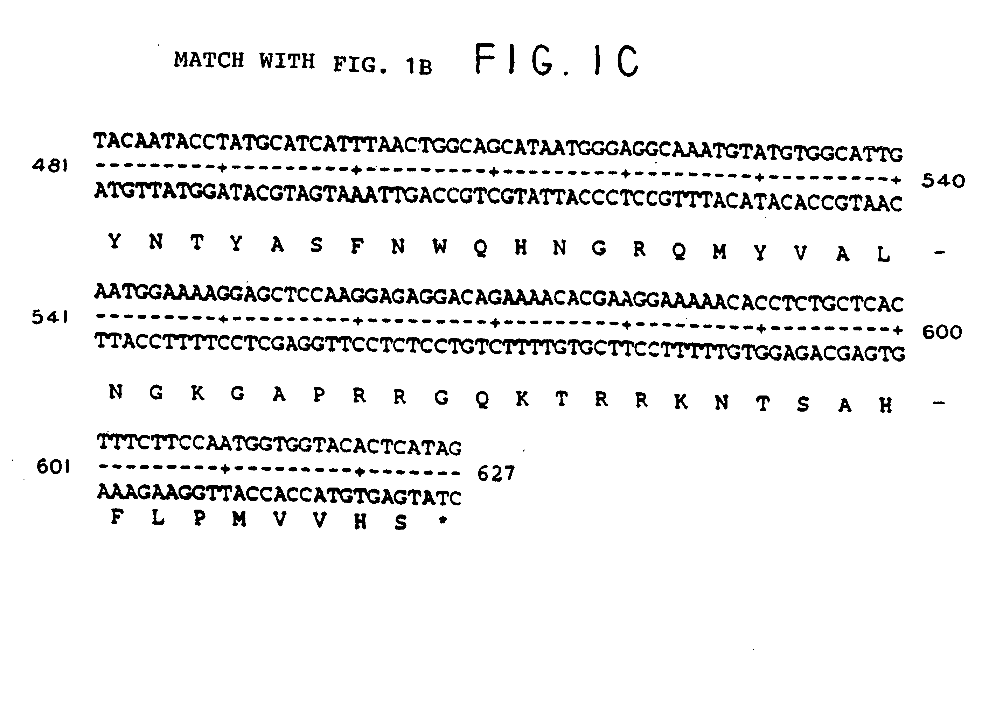

Figure 1 illustrates the cDNA and corresponding deduced amino acid sequence of the polypeptide of the present invention. The initial 36 amino acid residues represent the putative leader sequence (underlined). The standard one-letter abbreviations for amino acids are used. Sequencing inaccuracies are a common problem when attempting to determine polynucleotide sequences. Sequencing was performed using a 373 Automated DNA sequencer (Applied Biosystems, Inc.). Sequencing accuracy is predicted to be greater than 97% accurate.

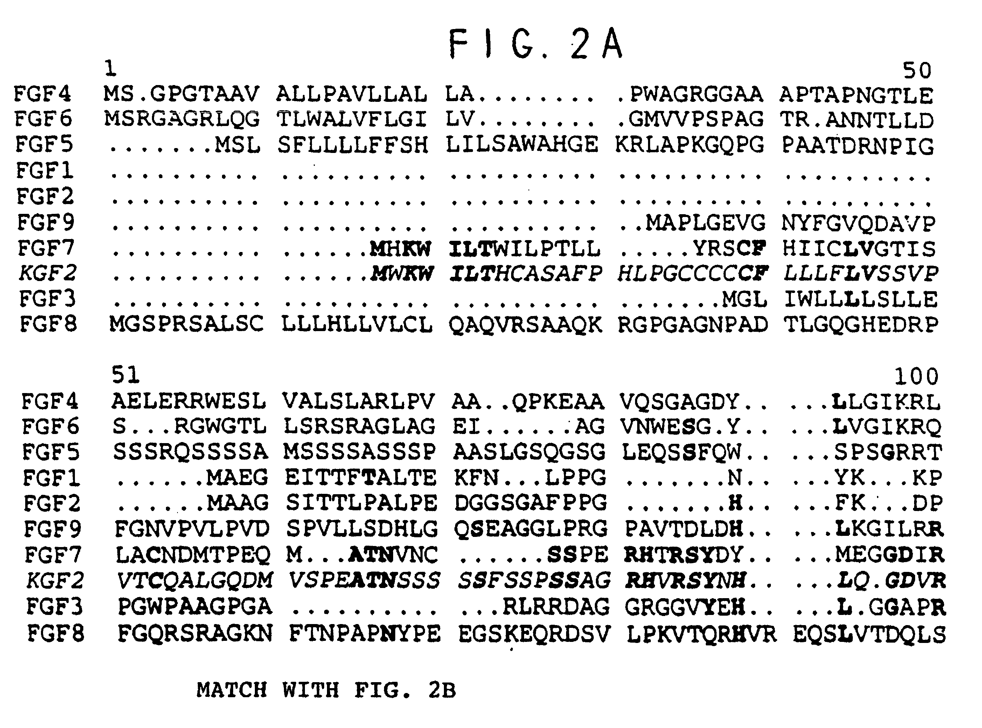

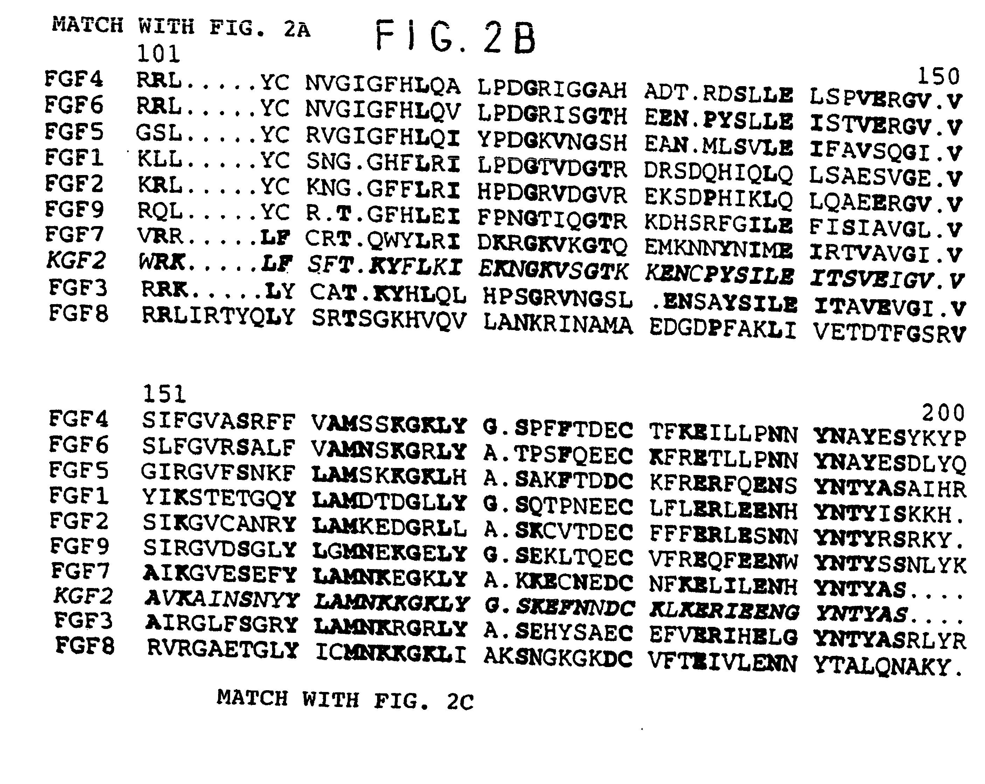

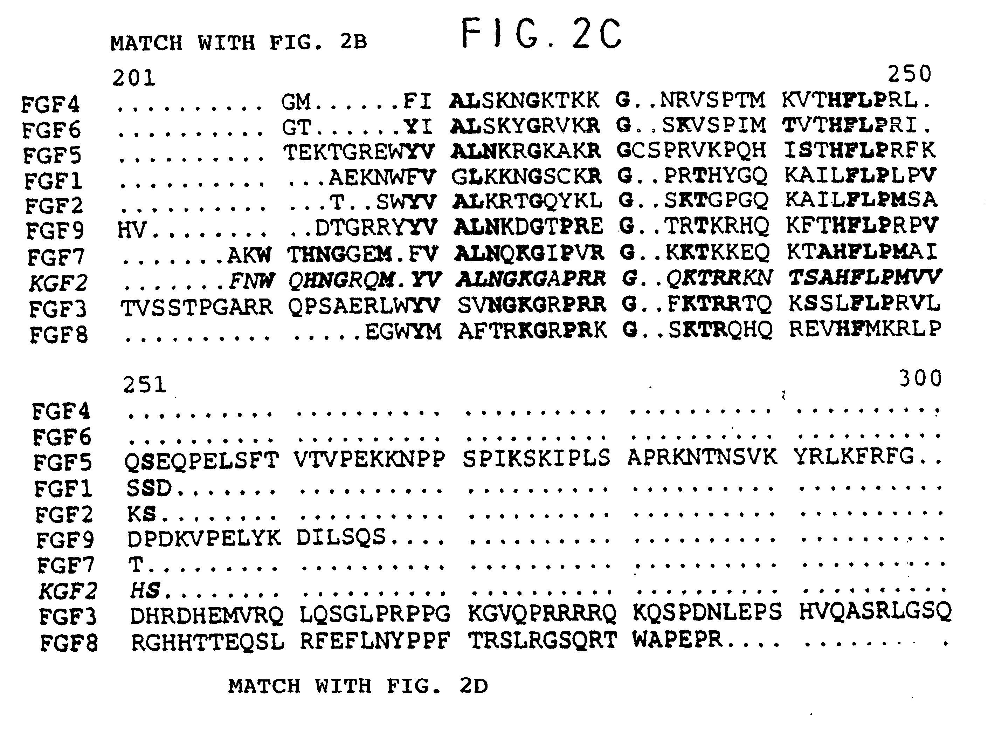

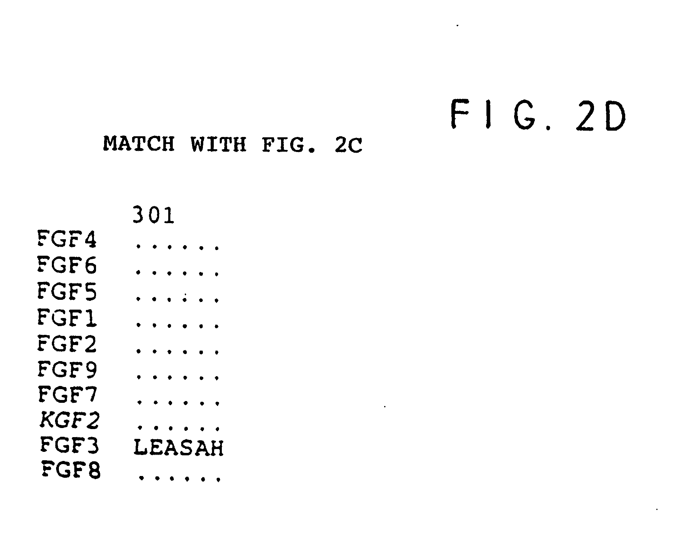

Figure 2 is an illustration of a comparison of the amino acid sequence of the polypeptide of the present invention and other fibroblast growth factors.

The present invention relates to a polynucleotide selected from the group consisting of
(a) polynucleotides comprising a nucleic acid sequence encoding amino acid residues -36 to +172 as set forth in SEQ ID NO: 2;
(b) polynucleotides comprising a nucleic acid sequence encoding amino acid residues +1 to +172 as set forth in SEQ ID NO:2;
(c) polynucleotides comprising a nucleic acid sequence encoding the full-length amino acid sequence encoded by the cDNA contained in ATCC 75977;
(d) polynucleotides comprising a nucleic acid sequence encoding the mature polypeptide encoded by the cDNA contained in ATCC 75977;
(e) polynucleotides comprising a nucleic acid sequence encoding a polypeptide fragment selected from the group consisting of:
   (i) a polypeptide fragment of amino acid residues -36 to +172 of SEQ ID NO:2;
   (ii) a polypeptide fragment of amino acid residues +1 to +172 of SEQ ID NO:2;
   (iii) a polypeptide fragment of the full-length polypeptide encoded by the cDNA contained in ATCC 75977; and
   (iv) a polypeptide fragment of the mature polypeptide encoded by the cDNA contained in ATCC 75977;
   wherein the polypeptide fragment stimulates proliferation of epithelial cells;
(f) polynucleotides comprising a first nucleic acid sequence which encodes a polypeptide which stimulates proliferation of epithelial cells and which is at least 95% identical to a second nucleotide sequence selected from the group consisting of:
   (i) a nucleotide sequence encoding amino acids +1 to +172 of SEQ ID NO:2;
   (ii) a nucleotide sequence encoding amino acids -36 to +172 of SEQ ID NO:2;
   (iii) a nucleotide sequence encoding a mature polypeptide encoded by the cDNA contained in ATCC 75977;
   (iv) a nucleotide sequence encoding the full-length polypeptide encoded by ATCC 75977;
   (v) a nucleotide sequence encoding a polypeptide fragment of amino acid residues -36 to +172 of SEQ ID NO:2, wherein said polypeptide fragment stimulates proliferation of epithelial cells;
   (vi) a nucleotide sequence encoding a polypeptide fragment of amino acid residues +1 to +172 of SEQ ID NO:2, wherein said polypeptide fragment stimulates proliferation of epithelial cells;
   (vii) a nucleotide sequence encoding a polypeptide fragment of the full-length polypeptide encoded by the cDNA contained in ATCC 75977, wherein said polypeptide fragment stimulates proliferation of epithelial cells; and
   (viii) a nucleotide sequence encoding a polypeptide fragment of the mature polypeptide encoded by the cDNA contained in ATCC 75977, wherein said polypeptide fragment stimulates proliferation of epithelial cells;
(g) the polynucleotide of (f), wherein said nucleotide sequence defined in any one of (i) to (viii) is selected from the group consisting of:
   (i) nucleotide sequences comprising nucleotides 109 to 627 of SEQ ID NO:1;
   (ii) nucleotide sequences comprising nucleotides 1 to 627 of SEQ ID NO:1;
   (iii) nucleotide sequences comprising the cDNA encoding the mature polypeptide encoded by the cDNA of ATCC 75977; and
   (iv) nucleotide sequences comprising the cDNA encoding the full-length polypeptide encoded by the cDNA of ATCC 75977;
(h) polynucleotides comprising a first nucleic acid sequence which encodes a polypeptide which stimulates proliferation of epithelial cells and which is at least 97% identical to a second nucleotide sequence selected from the group consisting of:
   (i) a nucleotide sequence encoding amino acids +1 to +172 of SEQ ID NO:2;
   (ii) a nucleotide sequence encoding amino acids -36 to +172 of SEQ ID NO:2;
   (iii) a nucleotide sequence encoding a mature polypeptide encoded by the cDNA contained in ATCC 75977;
   (iv) a nucleotide sequence encoding the full-length polypeptide encoded by ATCC 75977;
   (v) a nucleotide sequence encoding a polypeptide fragment of amino acid residues -36 to +172 of SEQ ID NO:2, wherein said polypeptide fragment stimulates proliferation of epithelial cells;
   (vi) a nucleotide sequence encoding a polypeptide fragment of amino acid residues +1 to +172 of SEQ ID NO:2, wherein said polypeptide fragment stimulates proliferation of epithelial cells;
   (vii) a nucleotide sequence encoding a polypeptide fragment of the full-length polypeptide encoded by the cDNA contained in ATCC 75977, wherein said polypeptide fragment stimulates proliferation of epithelial cells; and
   (viii) a nucleotide sequence encoding a polypeptide fragment of the mature polypeptide encoded by the cDNA contained in ATCC 75977, wherein said polypeptide fragment stimulates proliferation of epithelial cells;
(i) polynucleotides of at least 30 contiguous nucleotides of SEQ ID NO: 1;
(j) polynucleotides of at least 30 contiguous nucleotides of the cDNA contained in ATCC 75977;
(k) polynucleotides of 50 contiguous nucleotides of SEQ ID NO. 1; and
(l) polynucleotides of 50 contiguous nucleotides of the cDNA contained in ATCC 75977;
(m) polynucleotides of a greater number than 50 contiguous nucleotides of SEQ ID NO:1; and
(n) polynucleotides of a greater number than 50 contiguous nucleotides of the cDNA contained in ATCC 75977;
or the complementary strand of such a polynucleotide.

In accordance with an aspect of the present invention, there is provided a nucleic acid (polynucleotide) which encodes the mature polypeptide having the deduced amino acid sequence of Figure 1 (SEQ ID No. 2) or for the mature polypeptide encoded by the cDNA of the clone deposited as ATCC Deposit No. 75977 on December 16, 1994.

A polynucleotide encoding a polypeptide of the present invention may be obtained from a human prostate and fetal lung. A fragment of the cDNA encoding the polypeptide was initially isolated from a library derived from a human normal prostate. The open reading frame encoding the full length protein was subsequently isolated from a randomly primed human fetal lung cDNA library. It is structurally related to the FGF family. It contains an open reading frame encoding a protein of 208 amino acid residues of which approximately the first 36 amino acid residues are the putative leader sequence such that the mature protein comprises 172 amino acids. The protein exhibits the highest degree of homology to human keratinocyte growth factor with 45 % identity and 82 % similarity over a 206 amino acid stretch. It is also important that sequences that are conserved through the FGF family are found to be conserved in the protein of the present invention.

The polynucleotide of the present invention may be in the form of RNA or in the form of DNA, which DNA includes cDNA, genomic DNA, and synthetic DNA. The DNA may be double-stranded or single-stranded, and if single stranded may be the coding strand or non-coding (anti-sense) strand. The coding sequence which encodes the mature polypeptide may be identical to the coding sequence shown in Figure 1 (SEQ ID No. 1) or that of the deposited clone or may be a different coding sequence which coding sequence, as a result of the redundancy or degeneracy of the genetic code, encodes the same mature polypeptide as the DNA of Figure 1 (SEQ ID No. 1) or the deposited cDNA.

The polynucleotide which encodes for the mature polypeptide of Figure 1 (SEQ ID No. 2) or for the mature polypeptide encoded by the deposited cDNA may include: only the coding sequence for the mature polypeptide; the coding sequence for the mature polypeptide and additional coding sequence such as a leader or secretory sequence or a proprotein sequence; the coding sequence for the mature polypeptide (and optionally additional coding sequence) and non-coding sequence, such as introns or non-coding sequence 5' and/or 3' of the coding sequence for the mature polypeptide.

Thus, the term "polynucleotide encoding a polypeptide" encompasses a polynucleotide which includes only coding sequence for the polypeptide as well as a polynucleotide which includes additional coding and/or non-coding sequence.

The present invention further relates to variants of the hereinabove described polynucleotides which encode for fragments, analogs and derivatives of the polypeptide having the deduced amino acid sequence of Figure 1 (SEQ ID No. 2) or the polypeptide encoded by the cDNA of the deposited clone. The variant of the polynucleotide may be a naturally occurring allelic variant of the polynucleotide or a non-naturally occurring variant of the polynucleotide.

Thus, the present invention includes polynucleotides encoding the same mature polypeptide as shown in Figure 1 (SEQ ID No. 2) or the same mature polypeptide encoded by the cDNA of the deposited clone as well as variants of such polynucleotides which variants encode for a fragment, derivative or analog of the polypeptide of Figure 1 (SEQ ID No. 2) or the polypeptide encoded by the cDNA of the deposited clone. Such nucleotide variants include deletion variants, substitution variants and addition or insertion variants.

As hereinabove indicated, the polynucleotide may have a coding sequence which is a naturally occurring allelic variant of the coding sequence shown in Figure 1 (SEQ ID No. 1) or of the coding sequence of the deposited clone. As known in the art, an allelic variant is an alternate form of a polynucleotide sequence which may have a substitution, deletion or addition of one or more nucleotides, which does not substantially alter the function of the encoded polypeptide.

The present invention also includes polynucleotides, wherein the coding sequence for the mature polypeptide may be fused in the same reading frame to a polynucleotide sequence which aids in expression and secretion of a polypeptide from a host cell, for example, a leader sequence which functions as a secretory sequence for controlling transport of a polypeptide from the cell. The polypeptide having a leader sequence is a preprotein and may have the leader sequence cleaved by the host cell to form the mature form of the polypeptide. The polynucleotides may also encode for a proprotein which is the mature protein plus additional 5' amino acid residues. A mature protein having a prosequence is a proprotein and is an inactive form of the protein. Once the prosequence is cleaved an active mature protein remains.

Thus, for example, the polynucleotide of the present invention may encode for a mature protein, or for a protein having a prosequence or for a protein having both a prosequence and a presequence (leader sequence).

The polynucleotides of the present invention may also have the coding sequence fused in frame to a marker sequence which allows for purification of the polypeptide of the present invention. The marker sequence may be a hexahistidine tag supplied by a pQE-9 vector to provide for purification of the mature polypeptide fused to the marker in the case of a bacterial host, or, for example, the marker sequence may be a hemagglutinin (HA) tag when a mammalian host, e.g. COS-7 cells, is used. The HA tag corresponds to an epitope derived from the influenza hemagglutinin protein (Wilson, I., et al., Cell, 37:767 (1984)).

The present invention further relates to polynucleotides which hybridize to the hereinabove-described sequences if there is at least 50% and preferably 70% identity between the sequences. The present invention particularly relates to polynucleotides which hybridize under stringent conditions to the hereinabove-described polynucleotides. As herein used, the term "stringent conditions" means hybridization will occur only if there is at least 95 % and preferably at least 97 % identity between the sequences. The polynucleotides which hybridize to the hereinabove described polynucleotides in a preferred embodiment encode polypeptides which retain substantially the same biological function or activity as the mature polypeptide encoded by the cDNA of Figure 1 (SEQ ID No. 1) or the deposited cDNA.

The deposit(s) referred to herein will be maintained under the terms of the Budapest Treaty on the International Recognition of the Deposit of Micro-organisms for purposes of Patent Procedure. These deposits are provided merely as convenience to those of skill in the art and are not an admission that a deposit is required under 35 U.S.C. §112. The sequence of the polynucleotides contained in the deposited materials, as well as the amino acid sequence of the polypeptides encoded thereby, are incorporated herein by reference and are controlling in the event of any conflict with any description of sequences herein. A license may be required to make, use or sell the deposited materials, and no such license is hereby granted.

The present invention further relates to a polypeptide comprising an amino acid sequence encoded by a polynucleotide according to the invention, e.g. a polypeptide which has the deduced amino acid sequence of Figure 1 (SEQ ID No. 2) or which has the amino acid sequence encoded by the deposited cDNA, as well as fragments, analogs and derivatives of such polypeptide.

The terms "fragment," "derivative" and "analog" when referring to the polypeptide of Figure 1 (SEQ ID No. 2) or that encoded by the deposited cDNA, means a polypeptide which retains essentially the same biological function or activity as such polypeptide. Thus, an analog includes a proprotein which can be activated by cleavage of the proprotein portion to produce an active mature polypeptide.

The polypeptide of the present invention may be a recombinant polypeptide, a natural polypeptide or a synthetic polypeptide, preferably a recombinant polypeptide.

The fragment, derivative or analog of the polypeptide of Figure 1 (SEQ ID No. 2) or that encoded by the deposited cDNA may be (i) one in which one or more of the amino acid residues are substituted with a conserved or non-conserved amino acid residue (preferably a conserved amino acid residue) and such substituted amino acid residue may or may not be one encoded by the genetic code, or (ii) one in which one or more of the amino acid residues includes a substituent group, or (iii) one in which the mature polypeptide is fused with another compound, such as a compound to increase the half-life of the polypeptide (for example, polyethylene glycol), or (iv) one in which the additional amino acids are fused to the mature polypeptide, such as a leader or secretory sequence or a sequence which is employed for purification of the mature polypeptide or a proprotein sequence. Such fragments, derivatives and analogs are deemed to be within the scope of those skilled in the art from the teachings herein.

The polypeptides and polynucleotides of the present invention are preferably provided in an isolated form, and preferably are purified to homogeneity.

The term "isolated" means that the material is removed from its original environment (e.g., the natural environment if it is naturally occurring). For example, a naturally-occurring polynucleotide or polypeptide present in a living animal is not isolated, but the same polynucleotide or polypeptide, separated from some or all of the coexisting materials in the natural system, is isolated. Such polynucleotides could be part of a vector and/or such polynucleotides or polypeptides could be part of a composition, and still be isolated in that such vector or composition is not part of its natural environment.

The present invention also relates to vectors which include polynucleotides of the present invention, host cells which are genetically engineered with vectors of the invention and the production of polypeptides of the invention by recombinant techniques.

Host cells are genetically engineered (transduced or transformed or transfected) with the vectors of this invention which may be, for example, a cloning vector or an expression vector. The vector may be, for example, in the form of a plasmid, a viral particle, a phage, etc. The engineered host cells can be cultured in conventional nutrient media modified as appropriate for activating promoters, selecting transformants or amplifying the KGF-2 genes. The culture conditions, such as temperature, pH and the like, are those previously used with the host cell selected for expression, and will be apparent to the ordinarily skilled artisan.

The polynucleotides of the present invention may be employed for producing polypeptides by recombinant techniques. Thus, for example, the polynucleotide may be included in any one of a variety of expression vectors for expressing a polypeptide. Such vectors include chromosomal, nonchromosomal and synthetic DNA sequences, e.g., derivatives of SV40; bacterial plasmids; phage DNA; baculovirus; yeast plasmids; vectors derived from combinations of plasmids and phage DNA, viral DNA such as vaccinia, adenovirus, fowl pox virus, and pseudorabies. However, any other vector may be used as long as it is replicable and viable in the host.

The appropriate DNA sequence may be inserted into the vector by a variety of procedures. In general, the DNA sequence is inserted into an appropriate restriction endonuclease site(s) by procedures known in the art. Such procedures and others are deemed to be within the scope of those skilled in the art.

The DNA sequence in the expression vector is operatively linked to an appropriate expression control sequence(s) (promoter) to direct mRNA synthesis. As representative examples of such promoters, there may be mentioned: LTR or SV40 promoter, the E. coli. lac or trp, the phage lambda P_{L} promoter and other promoters known to control expression of genes in prokaryotic or eukaryotic cells or their viruses. The expression vector also contains a ribosome binding site for translation initiation and a transcription terminator. The vector may also include appropriate sequences for amplifying expression.

In addition, the expression vectors preferably contain one or more selectable marker genes to provide a phenotypic trait for selection of transformed host cells such as dihydrofolate reductase or neomycin resistance for eukaryotic cell culture, or such as tetracycline or ampicillin resistance in E. coli.

The vector containing the appropriate DNA sequence as hereinabove described, as well as an appropriate promoter or control sequence, may be employed to transform an appropriate host to permit the host to express the protein.

As representative examples of appropriate hosts, there may be mentioned: bacterial cells, such as E. coli, Streptomyces, Salmonella typhimurium; fungal cells, such as yeast; insect cells such as Drosophila S2 and Spodoptera Sf9; animal cells such as CHO, COS or Bowes melanoma; adenoviruses; plant cells, etc. The selection of an appropriate host is deemed to be within the scope of those skilled in the art from the teachings herein.

More particularly, the present invention also includes recombinant constructs comprising one or more of the sequences as broadly described above. The constructs comprise a vector, such as a plasmid or viral vector, into which a sequence of the invention has been inserted, in a forward or reverse orientation. In a preferred aspect of this embodiment, the construct further comprises regulatory sequences, including, for example, a promoter, operably linked to the sequence. Large numbers of suitable vectors and promoters are known to those of skill in the art, and are commercially available. The following vectors are provided by way of example; Bacterial: pQE70, pQE60, pQE-9 (Qiagen), pBS, pD10, phagescript, psiX174, pbluescript SK, pbsks, pNH8A, pNH16a, pNH18A, pNH46A (Stratagene) ; ptrc99a, pKK223-3, pKK233-3. pDR540, pRIT5 (Pharmacia); Eukaryotic: pWLNEO, pSV2CAT, pOG44, pXT1, pSG (Stratagene) pSVK3, pBPV, pMSG, pSVL (Pharmacia). However, any other plasmid or vector may be used as long as they are replicable and viable in the host.

Promoter regions can be selected from any desired gene using CAT (chloramphenicol transferase) vectors or other vectors with selectable markers. Two appropriate vectors are pKK232-8 and pCM7. Particular named bacterial promoters include lacI, lacZ, T3, T7, gpt, lambda P_{R}, P_{L} and trp. Eukaryotic promoters include CMV immediate early, HSV thymidine kinase, early and late SV40, LTRs from retrovirus, and mouse metallothionein-I. Selection of the appropriate vector and promoter is well within the level of ordinary skill in the art.

In a further embodiment, the present invention relates to host cells containing the above-described constructs. The host cell can be a higher eukaryotic cell, such as a mammalian cell, or a lower eukaryotic cell, such as a yeast cell, or the host cell can be a prokaryotic cell, such as a bacterial cell. Introduction of the construct into the host cell can be effected by calcium phosphate transfection, DEAE-Dextran mediated transfection, or electroporation (Davis, L., Dibner, M., Battey, I., Basic Methods in Molecular Biology, (1986)).

The constructs in host cells can be used in a conventional manner to produce the gene product encoded by the recombinant sequence. Alternatively, the polypeptides of the invention can be synthetically produced by conventional peptide synthesizers.

Mature proteins can be expressed in mammalian cells, yeast, bacteria, or other cells under the control of appropriate promoters. Cell-free translation systems can also be employed to produce such proteins using RNAs derived from the DNA constructs of the present invention. Appropriate cloning and expression vectors for use with prokaryotic and eukaryotic hosts are described by Sambrook, et al., Molecular Cloning: A Laboratory Manual, Second Edition, Cold Spring Harbor, N.Y., (1989), the disclosure of which is hereby incorporated by reference.

Transcription of the DNA encoding the polypeptides of the present invention by higher eukaryotes is increased by inserting an enhancer sequence into the vector. Enhancers are cis-acting elements of DNA, usually about from 10 to 300 bp that act on a promoter to increase its transcription. Examples including the SV40 enhancer on the late side of the replication origin bp 100 to 270, a cytomegalovirus early promoter enhancer, the polyoma enhancer on the late side of the replication origin, and adenovirus enhancers.

Generally, recombinant expression vectors will include origins of replication and selectable markers permitting transformation of the host cell, e.g., the ampicillin resistance gene of E. coli and S. cerevisiae TRP1 gene, and a promoter derived from a highly-expressed gene to direct transcription of a downstream structural sequence. Such promoters can be derived from operons encoding glycolytic enzymes such as 3-phosphoglycerate kinase (PGK), α-factor, acid phosphatase, or heat shock proteins, among others. The heterologous structural sequence is assembled in appropriate phase with translation initiation and termination sequences, and preferably, a leader sequence capable of directing secretion of translated protein into the periplasmic space or extracellular medium. Optionally, the heterologous sequence can encode a fusion protein including an N-terminal identification peptide imparting desired characteristics, e.g., stabilization or simplified purification of expressed recombinant product.

Useful expression vectors for bacterial use are constructed by inserting a structural DNA sequence encoding a desired protein together with suitable translation initiation and termination signals in operable reading phase with a functional promoter. The vector will comprise one or more phenotypic selectable markers and an origin of replication to ensure maintenance of the vector and to, if desirable, provide amplification within the host. Suitable prokaryotic hosts for transformation include E. coli, Bacillus subtilis, Salmonella typhimurium and various species within the genera Pseudomonas, Streptomyces, and Staphylococcus, although others may also be employed as a matter of choice.

As a representative but nonlimiting example, useful expression vectors for bacterial use can comprise a selectable marker and bacterial origin of replication derived from commercially available plasmids comprising genetic elements of the well known cloning vector pBR322 (ATCC 37017). Such commercial vectors include, for example, pKK223-3 (Pharmacia Fine Chemicals, Uppsala, Sweden) and GEM1 (Promega Biotec, Madison, WI, USA). These pBR322 "backbone" sections are combined with an appropriate promoter and the structural sequence to be expressed.

Following transformation of a suitable host strain and growth of the host strain to an appropriate cell density, the selected promoter is induced by appropriate means (e.g., temperature shift or chemical induction) and cells are cultured for an additional period.

Cells are typically harvested by centrifugation, disrupted by physical or chemical means, and the resulting crude extract retained for further purification.

Microbial cells employed in expression of proteins can be disrupted by any convenient method, including freeze-thaw cycling, sonication, mechanical disruption, or use of cell lysing agents, such methods are well know to those skilled in the art.

Various mammalian cell culture systems can also be employed to express recombinant protein. Examples of mammalian expression systems include the COS-7 lines of monkey kidney fibroblasts, described by Gluzman, Cell, 23:175 (1981), and other cell lines capable of expressing a compatible vector, for example, the C127, 3T3, CHO, HeLa and BHK cell lines. Mammalian expression vectors will comprise an origin of replication, a suitable promoter and enhancer, and also any necessary ribosome binding sites, polyadenylation site, splice donor and acceptor sites, transcriptional termination sequences, and 5' flanking nontranscribed sequences. DNA sequences derived from the SV40 splice, and polyadenylation sites may be used to provide the required nontranscribed genetic elements.

The KGF-2 polypeptide can be recovered and purified from recombinant cell cultures by methods including ammonium sulfate or ethanol precipitation, acid extraction, anion or cation exchange chromatography, phosphocellulose chromatography, hydrophobic interaction chromatography, affinity chromatography, hydroxylapatite chromatography and lectin chromatography. Protein refolding steps can be used, as necessary, in completing configuration of the mature protein. Finally, high performance liquid chromatography (HPLC) can be employed for final purification steps.

The polypeptides of the present invention may be a naturally purified product, or a product of chemical synthetic procedures, or produced by recombinant techniques from a prokaryotic or eukaryotic host (for example, by bacterial, yeast, higher plant, insect and mammalian cells in culture). Depending upon the host employed in a recombinant production procedure, the polypeptides of the present invention may be glycosylated or may be non-glycosylated. Polypeptides of the invention may also include an initial methionine amino acid residue.

The polypeptide of the present invention may be useful for stimulating new blood vessel growth or angiogenesis. Particularly, the polypeptide of the present invention may stimulate keratinocyte cell growth and proliferation. Accordingly, the polypeptide of the present invention may be used for the preparation of a pharmaceutical composition for stimulating wound healing, and also for stimulating Keratinocytes which is related to the prevention of hair loss.

The polypeptide of the present invention may also be useful for healing dermal wounds by stimulating epithelial cell proliferation.

The polypeptide of the present invention may also be useful for stimulating differentiation of cells, for example, muscle cells and nervous tissue, prostate cells and lung cells.

The signal sequence of KGF-2 encoding amino acids 1 through 36 may be employed to identify secreted proteins in general by hybridization and/or computational search algorithms.

The nucleotide sequence of KGF-2 could be employed to isolate 5' sequences by hybridization. Plasmids comprising the KGF-2 gene under the control of its native promoter/enhancer sequences could then be used in *in vitro* studies aimed at the identification of endogenous cellular and viral transactivators of KGF-2 gene expression.

The KGF-2 protein may also be employed as a positive control in experiments designed to identify peptido-mimetics acting upon the KGF-2 receptor.

The polynucleotides or polypeptides according to the invention can also be used in a process for *in vitro* purposes related to scientific research, synthesis of DNA, manufacture of DNA vectors and for the purpose of providing diagnostics and therapeutics for the treatment of human disease.

Fragments of the full length, KGF-2 gene may be used as a hybridization probe for a cDNA library to isolate the full length KGF-2 genes and to isolate other genes which have a high sequence similarity to these genes or similar biological activity. Probes of this type generally have at least 20 bases. Preferably, however, the probes have at least 30 bases and generally do not exceed 50 bases, although they may have a greater number of bases. The probe may also be used to identify a cDNA clone corresponding to a full length transcript and a genomic clone or clones that contain the complete KGF-2 gene including regulatory and promoter regions, exons, and introns. An example of a screen comprises isolating the coding region of the KGF-2 gene by using the known DNA sequence to synthesize an oligonucleotide probe. Labeled oligonucleotides having a sequence complementary to that of the gene of the present invention are used to screen a library of human cDNA, genomic DNA or mRNA to determine which members of the library the probe hybridizes to.

The polynucleotides or polypeptides according to the invention can be employed in a method for identification of the receptors for the KGF-2 polypeptide. The gene encoding the receptor can be identified by numerous methods known to those of skill in the art, for example, ligand panning and FACS sorting (Coligan, et al., Current Protocols in Immun., 1 (2), Chapter 5, (1991)). Preferably, expression cloning is employed wherein polyadenylated RNA is prepared from a cell responsive to the polypeptides, and a cDNA library created from this RNA is divided into pools and used to transfect COS cells or other cells that are not responsive to the polypeptides. Transfected cells which are grown on glass slides are exposed to the labeled polypeptides. The polypeptides can be labeled by a variety of means including iodination or inclusion of a recognition site for a site-specific protein kinase. Following fixation and incubation, the slides are subjected to autoradiographic analysis. Positive pools are identified and sub-pools are prepared and re-transfected using an iterative sub-pooling and re-screening process, eventually yielding a single clones that encodes the putative receptor.

As an alternative approach for receptor identification, the labeled polypeptides can be photoaffinity linked with cell membrane or extract preparations that express the receptor molecule. Cross-linked material is resolved by PAGE analysis and exposed to x-ray film. The labeled complex containing the receptors of the polypeptides can be excised, resolved into peptide fragments, and subjected to protein microsequencing. The amino acid sequence obtained from microsequencing would be used to design a set of degenerate oligonucleotide probes to screen a cDNA library to identify the genes encoding the putative receptors.

The polynucleotides or polypeptides according to the invention can be employed in a method of screening compounds to identify those which agonize the action of KGF-2 or block the function of KGF-2. An example of such an assay comprises combining a mammalian Keratinocyte cell, the compound to be screened and ³[H] thymidine under cell culture conditions where the keratinocyte cell would normally proliferate. A control assay may be performed in the absence of the compound to be screened and compared to the amount of keratinocyte proliferation in the presence of the compound to determine if the compound stimulates proliferation of Keratinocytes.

To screen for antagonists, the same assay may be prepared in the presence of KGF-2 and the ability of the compound to prevent Keratinocyte proliferation is measured and a determination of antagonist ability is made. The amount: of Keratinocyte cell proliferation is measured by liquid scintillation chromatography which measures the incorporation of ³[H] thymidine.

In another method, a mammalian cell or membrane preparation expressing the KGF-2 receptor would be incubated with labeled KGF-2 in the presence of the compound. The ability of the compound to enhance or block this interaction could then be measured. Alternatively, the response of a known second messenger system following interaction of KGF-2 and receptor would be measured and compared in the presence or absence of the compound. Such second messenger systems include but are not limited to, cAMP guanylate cyclase, ion channels or phosphoinositide hydrolysis.

Examples of potential KGF-2 antagonists include an antibody, or in some cases, an oligonucleotide, which binds to the polypeptide. Alternatively, a potential KGF-2 antagonist may be a mutant form of KGF-2 which binds to KGF-2 receptors, however, no second messenger response is elicited and therefore the action of KGF-2 is effectively blocked.

Another potential KGF-2 antagonist is an antisense construct prepared using antisense technology. Antisense technology can be used to control gene expression through triple-helix formation or antisense DNA or RNA, both of which methods are based on binding of a polynucleotide to DNA or RNA. For example, the 5' coding portion of the polynucleotide sequence, which encodes for the mature polypeptides of the present invention, is used to design an antisense RNA oligonucleotide of from about 10 to 40 base pairs in length. A DNA oligonucleotide is designed to be complementary to a region of the gene involved in transcription (triple helix -see Lee et al., Nucl. Acids Res., 6:3073 (1979); Cooney et al, Science, 241:456 (1988); and Dervan et al., Science, 251: 1360 (1991)), thereby preventing transcription and the production of KGF-2. The antisense RNA oligonucleotide hybridizes to the mRNA *in vivo* and blocks translation of the mRNA molecule into KGF-2 polypeptide (Antisense - Okano, J. Neurochem., 56:560 (1991); Oligodeoxynucleotides as Antisense Inhibitors of Gene Expression, CRC Press, Boca Raton, FL (1988)). The oligonucleotides described above can also be delivered to cells such that the antisense RNA or DNA may be expressed *in vivo* to inhibit production of KGF-2.

Potential KGF-2 antagonists include small molecules which bind to and occupy the binding site of the KGF-2 receptor thereby making the receptor inaccessible to KGF-2 such that normal biological activity is prevented. Examples of small molecules include but are not limited to small peptides or peptide-like molecules.

The KGF-2 antagonists may be employed to prevent the induction of new blood vessel grown or angiogenesis in tumors. Angiogenesis stimulated by KGF-2 also contributes to several pathologies which may also be treated by the antagonists of the present invention, including diabetic retinopathy, and inhibition of the growth of pathological tissues, such as in rheumatoid arthritis.

KGF-2 antagonists may also be employed to treat glomerulonephritis, which is characterized by the marked proliferation of glomerular epithelial cells which form a cellular mass filling Bowman's space.

The antagonists may also be useful for inhibiting the over-production of scar tissue seen in keloid formation after surgery, fibrosis after myocardial infarction or fibrotic lesions associated with pulmonary fibrosis and restenosis. KGF-2 antagonists may also be employed to treat other proliterative diseases which are stimulated by KGF-2, including cancer and Kaposi's sarcoma.

KGF-2 antagonists may also be useful for treating keratitis which is a chronic infiltration of the deep layers of the cornea with uveal inflammation characterized by epithelial cell proliferation.

The antagonists may be employed in a composition with a pharmaceutically acceptable carrier, e.g., as hereinafter described.

The polypeptides, agonists and antagonists of the present invention may be employed in combination with a suitable pharmaceutical carrier to comprise a pharmaceutical composition. Such compositions comprise a therapeutically effective amount of the polypeptide, agonist or antagonist and a pharmaceutically acceptable carrier or excipient. Such a carrier includes but is not limited to saline, buffered saline, dextrose, water, glycerol, ethanol, and combinations thereof. The formulation should suit the mode of administration.

The invention also provides a pharmaceutical pack or kit comprising one or more containers filled with one or more of the ingredients of the pharmaceutical compositions of the invention. Associated with such container(s) can be a notice in the form prescribed by a governmental agency regulating the manufacture, use or sale of pharmaceuticals or biological products, which notice reflects approval by the agency of manufacture, use or sale for human administration. In addition, the polypeptides, agonists and antagonists of the present invention may be employed in conjunction with other therapeutic compounds.

The pharmaceutical compositions may be administered in a convenient manner such as by the oral, topical, intravenous, intraperitoneal, intramuscular, subcutaneous, intranasal or intradermal routes. The pharmaceutical compositions are administered in an amount which is effective for treating and/or prophylaxis of the specific indication. In general, they are administered in an amount of at least about 10 µg/kg body weight and in most cases they will be administered in an amount not in excess of about 8 mg/Kg body weight per day. In most cases, the dosage is from about 10 µg/kg to about 1 mg/kg body weight daily, taking into account the routes of administration, symptoms, etc. In the specific case of topical administration dosages are preferably administered from about 0.1 µg to 9 mg per cm².

The KGF-2 polypeptides, agonists and antagonists which are polypeptides may also be useful in accordance with the present invention for expressing such polypeptides *in vivo*, which is often referred to as "gene therapy."

Thus, for example, cells from a patient may be engineered with a polynucleotide (DNA or RNA) encoding a polypeptide *ex vivo*, with the engineered cells then being provided to a patient to be treated with the polypeptide. Such methods are well-known in the art. For example, cells may be engineered by procedures known in the art by use of a retroviral particle containing RNA encoding a polypeptide of the present invention.

Similarly, cells may be engineered *in vivo* for expression of a polypeptide *in vivo* by, for example, procedures known in the art. As known in the art, a producer cell for producing a retroviral particle containing RNA encoding the polypeptide of the present invention may be administered to a patient for engineering cells *in vivo* and expression of the polypeptide *in vivo*. These and other methods for administering a polypeptide of the present invention by such method should be apparent to those skilled in the art from the teachings of the present invention. For example, the expression vehicle for engineering cells may be other than a retrovirus, for example, an adenovirus which may be used to engineer cells in vivo after combination with a suitable delivery vehicle. Examples of other delivery vehicles include an HSV-based vector system, adeno-associated virus vectors, and inert vehicles, for example, dextran coated ferrite particles. The polynucleotides according to the invention can also be used as part of a diagnostic assay for detecting diseases or susceptibility to diseases related to the presence of mutations in the KGF-2 nucleic acid sequences.

Individuals carrying mutations in the KGF-2 gene may be detected at the DNA level by a variety of techniques. Nucleic acids for diagnosis may be obtained from a patient's cells, such as from blood, urine, saliva, tissue biopsy and autopsy material. The genomic DNA may be used directly for detection or may be amplified enzymatically by using PCR (Saiki *et al.*, Nature, 324:163-166 (1986)) prior to analysis. RNA or cDNA may also be used for the same purpose. As an example, PCR primers complementary to the nucleic acid encoding KGF-2 can be used to identify and analyze KGF-2 mutations. For example, deletions and insertions can be detected by a change in size of the amplified product in comparison to the normal genotype. Point mutations can be identified by hybridizing amplified DNA to radiolabeled KGF-2 RNA or alternatively, radiolabeled KGF-2 antisense DNA sequences. Perfectly matched sequences can be distinguished from mismatched duplexes by RNase A digestion or by differences in melting temperatures.

Genetic testing based on DNA sequence differences may be achieved by detection of alteration in electrophoretic mobility of DNA fragments in gels with or without denaturing agents. Small sequence deletions and insertions can be visualized by high resolution gel electrophoresis. DNA fragments of different sequences may be distinguished on denaturing formamide gradient gels in which the mobilities of different DNA fragments are retarded in the gel at different positions according to their specific melting or partial melting temperatures (see, e.g., Myers *et al*., Science, 230:1242 (1985)).

Sequence changes at specific locations may also be revealed by nuclease protection assays, such as RNase and S1 protection or the chemical cleavage method (e.g., Cotton *et al*., PNAS, USA, 85:4397-4401 (1985)).

Thus, the detection of a specific DNA sequence may be achieved by methods such as hybridization, RNase protection, chemical cleavage, direct DNA sequencing or the use of restriction enzymes, (e.g., Restriction Fragment Length Polymorphisms (RFLP)) and Southern blotting of genomic DNA.

In addition to more conventional gel-electrophoresis and DNA sequencing, mutations can also be detected by *in situ* analysis.

The polynucleotides, polypeptides or antibodies according to the invention may be employed in a diagnostic assay for detecting altered levels of KGF-2 protein in various tissues since an over-expression of the proteins compared to normal control tissue samples may detect the presence of a disease or susceptibility to a disease, for example, a tumor. Assays used to detect levels of KGF-2 protein in a sample derived from a host are well-known to those of skill in the art and include radioimmunoassays, competitive-binding assays, Western Blot analysis, ELISA assays and "sandwich" assay. An ELISA assay (Coligan, et al., Current Protocols in Immunology, 1(2), Chapter 6, (1991)) initially comprises preparing an antibody specific to the KGF-2 antigen, preferably a monoclonal antibody. In addition a reporter antibody is prepared against the monoclonal antibody. To the reporter antibody is attached a detectable reagent such as radioactivity, fluorescence or, in this example, a horseradish peroxidase enzyme. A sample is removed from a host and incubated on a solid support, e.g. a polystyrene dish, that binds the proteins in the sample. Any free protein binding sites on the dish are then covered by incubating with a non-specific protein like bovine serum albumen. Next, the monoclonal antibody is incubated in the dish during which time the monoclonal antibodies attach to any KGF-2 proteins attached to the polystyrene dish. All unbound monoclonal antibody is washed out with buffer. The reporter antibody linked to horseradish peroxidase is now placed in the dish resulting in binding of the reporter antibody to any monoclonal antibody bound to KGF-2. Unattached reporter antibody is then washed out. Peroxidase substrates are then added to the dish and the amount of color developed in a given time period is a measurement of the amount of KGF-2 protein present in a given volume of patient sample when compared against a standard curve.

A competition assay may be employed wherein antibodies specific to KGF-2 are attached to a solid support and labeled KGF-2 and a sample derived from the host are passed over the solid support and the amount of label detected, for example by liquid scintillation chromatography, can be correlated to a quantity of KGF-2 in the sample.

A "sandwich" assay is similar to an ELISA assay. In a "sandwich" assay KGF-2 is passed over a solid support and binds to antibody attached to a solid support. A second antibody is then bound to the KGF-2. A third antibody which is labeled and specific to the second antibody is then passed over the solid support and binds to the second antibody and an amount can then be quantified.

The sequences of the present invention are also valuable for chromosome identification. The sequence is specifically targeted to and can hybridize with a particular location on an individual human chromosome. Moreover, there is a current need for identifying particular sites on the chromosome. Few chromosome marking reagents based on actual sequence data (repeat polymorphisms) are presently available for marking chromosomal location. The mapping of DNAs to chromosomes according to the present invention is an important first step in correlating those sequences with genes associated with disease.

Briefly, sequences can be mapped to chromosomes by preparing PCR primers (preferably 15-25 bp) from the cDNA. Computer analysis of the 3' untranslated region is used to rapidly select primers that do not span more than one exon in the genomic DNA, thus complicating the amplification process. These primers are then used for PCR screening of somatic cell hybrids containing individual human chromosomes. Only those hybrids containing the human gene corresponding to the primer will yield an amplified fragment.

PCR mapping of somatic cell hybrids is a rapid procedure for assigning a particular DNA to a particular chromosome. Using the present invention with the same oligonucleotide primers, sublocalization can be achieved with panels of fragments from specific chromosomes or pools of large genomic clones in an analogous manner. Other mapping strategies that can similarly be used to map to its chromosome include *in situ* hybridization, prescreening with labeled flow-sorted chromosomes and preselection by hybridization to construct chromosome specific-cDNA libraries.

Fluorescence *in situ* hybridization (FISH) of a cDNA clone to a metaphase chromosomal spread can be used to provide a precise chromosomal location in one step. This technique can be used with cDNA as short as 500 or 600 bases; however, clones larger than 2,000 bp have a higher likelihood of binding to a unique chromosomal location with sufficient signal intensity for simple detection. FISH requires use of the clones from which the EST was derived, and the longer the better. For example, 2,000 bp is good, 4,000 is better, and more than 4,000 is probably not necessary to get good results a reasonable percentage of the time. For a review of this technique, see Verma et al., Human Chromosomes: a Manual of Basic Techniques, Pergamon Press, New York (1988).

Once a sequence has been mapped to a precise chromosomal location, the physical position of the sequence on the chromosome can be correlated with genetic map data. Such data are found, for example, in V. McKusick, Mendelian Inheritance in Man (available on line through Johns Hopkins University Welch Medical Library). The relationship between genes and diseases that have been mapped to the same chromosomal region are then identified through linkage analysis (coinheritance of physically adjacent genes).

Next, it is necessary to determine the differences in the cDNA or genomic sequence between affected and unaffected individuals. If a mutation is observed in some or all of the affected individuals but not in any normal individuals, then the mutation is likely to be the causative agent of the disease.

With current resolution of physical mapping and genetic mapping techniques, a cDNA precisely localized to a chromosomal region associated with the disease could be one of between 50 and 500 potential causative genes. (This assumes 1 megabase mapping resolution and one gene per 20 kb).

The polypeptides, their fragments or other derivatives, or analogs thereof, or cells expressing them can be used as an immunogen to produce antibodies thereto. These antibodies can be, for example, polyclonal or monoclonal antibodies. The present invention also includes chimeric, single chain, and humanized antibodies, as well as Fab fragments, or the product of an Fab expression library. Various procedures known in the art may be used for the production of such antibodies and fragments.

Antibodies generated against the polypeptides corresponding to a sequence of the present invention can be obtained by direct injection of the polypeptides into an animal or by administering the polypeptides to an animal, preferably a nonhuman. The antibody so obtained will then bind the polypeptides itself. In this manner, even a sequence encoding only a fragment of the polypeptides can be used to generate antibodies binding the whole native polypeptides. Such antibodies can then be used to isolate the polypeptide from tissue expressing that polypeptide.

For preparation of monoclonal antibodies, any technique which provides antibodies produced by continuous cell line cultures can be used. Examples include the hybridoma technique (Kohler and Milstein, 1975, Nature, 256:495-497), the trioma technique, the human B-cell hybridoma technique (Kozbor et al., 1983, Immunology Today 4:72), and the EBV-hybridoma technique to produce human monoclonal antibodies (Cole, et al., 1985, in Monoclonal Antibodies and Cancer Therapy, Alan R. Liss, Inc., pp. 77-96).

Techniques described for the production of single chain antibodies (U.S. Patent 4,946,778) can be adapted to produce single chain antibodies to immunogenic polypeptide products of this invention. Also, transgenic mice may be used to express humanized antibodies to immunogenic polypeptide products of this invention.

The present invention will be further described with reference to the following examples; however, it is to be understood that the present invention is not limited to such examples. All parts or amounts, unless otherwise specified, are by weight.

In order to facilitate understanding of the following examples certain frequently occurring methods and/or terms will be described.

"Plasmids" are designated by a lower case p preceded and/or followed by capital letters and/or numbers. The starting plasmids herein are either commercially available, publicly available on an unrestricted basis, or can be constructed from available plasmids in accord with published procedures. In addition, equivalent plasmids to those described are known in the art and will be apparent to the ordinarily skilled artisan.

"Digestion" of DNA refers to catalytic cleavage of the DNA with a restriction enzyme that acts only at certain sequences in the DNA. The various restriction enzymes used herein are commercially available and their reaction conditions, cofactors and other requirements were used as would be known to the ordinarily skilled artisan. For analytical purposes, typically 1 µg of plasmid or DNA fragment is used with about 2 units of enzyme in about 20 µl of buffer solution. For the purpose of isolating DNA fragments for plasmid construction, typically 5 to 50 µg of DNA are digested with 20 to 250 units of enzyme in a larger volume. Appropriate buffers and substrate amounts for particular restriction enzymes are specified by the manufacturer. Incubation times of about 1 hour at 37°C are ordinarily used, but may vary in accordance with the supplier's instructions. After digestion the reaction is electrophoresed directly on a polyacrylamide gel to isolate the desired fragment.

Size separation of the cleaved fragments is performed using 8 percent polyacrylamide gel described by Goeddel, D. *et al*., Nucleic Acids Res., 8:4057 (1980).

"Oligonucleotides" refers to either a single stranded polydeoxynucleotide or two complementary polydeoxynucleotide strands which may be chemically synthesized. Such synthetic oligonucleotides have no 5' phosphate and thus will not ligate to another oligonucleotide without adding a phosphate with an ATP in the presence of a kinase. A synthetic oligonucleotide will ligate to a fragment that has not been dephosphorylated.

"Ligation" refers to the process of forming phosphodiester bonds between two double stranded nucleic acid fragments (Maniatis, T., et al., Id., p. 146). Unless otherwise provided, ligation may be accomplished using known buffers and conditions with 10 units of T4 DNA ligase ("ligase") per 0.5 µg of approximately equimolar amounts of the DNA fragments to be ligated.

A cell has been "transformed" by exogenous DNA when such exogenous DNA has been introduced inside the cell membrane. Exogenous DNA may or may not be integrated (covalently linked) inter-chromosomal DNA making the genome of the cell. Prokaryote and yeast, for example, the exogenous DNA may be maintained on an episomal element, such a plasmid. With respect to eukaryotic cells, a stably transformed or transfected cell is one in which the exogenous DNA has become integrated into the chromosome so that it is inherited by daughter cells through chromosome replication. This ability is demonstrated by the ability of the eukaryotic cell to establish cell lines or clones comprised of a population of daughter cell containing the exogenous DNA. An example of transformation is exhibited in Graham, F. and Van der Eb, A., Virology, 52:456-457 (1973).

"Transduction" or "transduced" refers to a process by which cells take up foreign DNA and integrate that foreign DNA into their chromosome. Transduction can be accomplished, for example, by transfection, which refers to various techniques by which cells take up DNA, or infection, by which viruses are used to transfer DNA into cells.

### Example 1

### Bacterial Expression and Purification of KGF-2

The DMA sequence encoding KGF-2, ATCC # 75977, is initially amplified using PCR oligonucleotide primers corresponding to the 5' and 3' end sequences of the processed KGF-2 cDNA (including the signal peptide sequence). The 5' oligonucleotide primer has the sequence 5' CCCCACATGTGGAAATGGATACTGACACATTGTGCC 3' (SEQ ID No. 3) contains an Afl III restriction enzyme site including and followed by 30 nucleotides of KGF-2 coding sequence starting from the presumed initiation codon. The 3' sequence 5' CCCAAGCTTCCACAAACGTTGCCTTCCTCTATGAG 3' (SEQ ID No. 4) contains complementary sequences to Hind III site and is followed by 26 nucleotides of KGF-2. The restriction enzyme sites are compatible with the restriction enzyme sites on the bacterial expression vector pQE-60 (Qiagen, Inc. Chatsworth, CA). pQE-60 encodes antibiotic resistance (Amp^{r}), a bacterial origin of replication (ori), an IPTG-regulatable promoter operator (P/O), a ribosome binding site (RBS), a 6-His tag and restriction enzyme sites. pQE-60 is then digested with NcoI and HindIII. The amplified sequences are ligated into pQE-60 and are inserted in frame. The ligation mixture is then used to transform E. coli strain M15/rep 4 (Qiagen, Inc.) by the procedure described in Sambrook, J. et al., Molecular Cloning: A Laboratory Manual, Cold Spring Laboratory Press, (1989). M15/rep4 contains multiple copies of the plasmid pREP4, which expresses the lacI repressor and also confers kanamycin resistance (Kan^{r}). Transformants are identified by their ability to grow on LB plates and ampicillin/kanamycin resistant colonies are selected. Plasmid DNA is isolated and confirmed by restriction analysis. Clones containing the desired constructs are grown overnight (O/N) in liquid culture in LB media supplemented with both Amp (100 ug/ml) and Kan (25 ug/ml). The O/N culture is used to inoculate a large culture at a ratio of 1:100 to 1:250. The cells are grown to an optical density 600 (O.D.⁶⁰⁰) of between 0.4 and 0.6. IPTG ("Isopropyl-B-D-thiogalacto pyranoside") is then added to a final concentration of 1 mM. IPTG induces by inactivating the lacI repressor, clearing the P/O leading to increased gene expression. Cells are grown an extra 3 to 4 hours. Cells are then harvested by centrifugation. The cell pellet is solubilized in the chaotropic agent 6 Molar Guanidine HCl. After clarification, solubilized KGF-2 is purified from this solution by chromatography on a Heparin affinity column under conditions that allow for tight binding of the proteins (Hochuli, E. et al., J. Chromatography 411:177-184 (1984)). KGF-2 ( 75 % pure) is eluted from the column by high salt buffer.

### Example 2

### Bacterial Expression and Purification of a truncated version of KGF-2

The DNA sequence encoding KGF-2, ATCC # 75977, is initially amplified using PCR oligonucleotide primers corresponding to the 5' and 3' sequences of the truncated version of the KGF-2 polypeptide. The truncated version comprises the polypeptide minus the 36 amino acid signal sequence, with a methionine and alanine residue being added just before the cysteine residue which comprises amino acid 37 of the full-length protein. The 5' oligonucleotide primer has the sequence 5' CATGCCATGGCGTGCCAAGCCCTTGGTCAGGACATG 3' (SEQ ID No. 5) contains an NcoI restriction enzyme site including and followed by 24 nucleotides of KGF-2 coding sequence. The 3' sequence 5' CCCAAGCTTCCACAAACGTTGCCTTCCTC TATGAG 3' (SEQ ID No. 6) contains complementary sequences to Hind III site and is followed by 26 nucleotides of the KGF-2 gene. The restriction enzyme sites are compatible with the restriction enzyme sites on the bacterial expression vector pQE-60 (Qiagen, Inc. Chatsworth, CA). pQE-60 encodes antibiotic resistance (Amp^{r}), a bacterial origin of replication (ori), an IPTG-regulatable promoter operator (P/O), a ribosome binding site (RBS), a 6-His tag and restriction enzyme sites. pQE-60 is then digested with NcoI and HindIII. The amplified sequences are ligated into pQE-60 and are inserted in frame. The ligation mixture is then used to transform E. coli strain M15/rep 4 (Qiagen, Inc.) by the procedure described in Sambrook, J. et al., Molecular Cloning: A Laboratory Manual, Cold Spring Laboratory Press, (1989). M15/rep4 contains multiple copies of the plasmid pREP4, which expresses the lacI repressor and also confers kanamycin resistance (Kan^{r}). Transformants are identified by their ability to grow on LB plates and ampicillin/kanamycin resistant colonies are selected. Plasmid DNA is isolated and confirmed by restriction analysis. Clones containing the desired constructs are grown overnight (O/N) in liquid culture in LB media supplemented with both Amp (100 ug/ml) and Kan (25 ug/ml). The O/N culture is used to inoculate a large culture at a ratio of 1:100 to 1:250. The cells are grown to an optical density 600 (O.D.⁶⁰⁰) of between 0.4 and 0.6. IPTG ("Isopropyl-B-D-thiogalacto pyranoside") is then added to a final concentration of 1 mM. IPTG induces by inactivating the lacI repressor, clearing the P/O leading to increased gene expression. Cells are grown an extra 3 to 4 hours. Cells are then harvested by centrifugation. The cell pellet is solubilized in the chaotropic agent 6 Molar Guanidine HCl. After clarification, solubilized KGF-2 is purified from this solution by chromatography on a Heparin affinity column under conditions that allow for tight binding the proteins (Hochuli, E. et al., J. Chromatography 411:177-184 (1964)). KGF-2 protein is eluted from the column by high salt buffer.

### Example 3

### Cloning and expression of KGF-2 using the baculovirus expression system

The DNA sequence encoding the full length KGF-2 protein, ATCC # 75977, is amplified using PCR oligonucleotide primers corresponding to the 5' and 3' sequences of the gene:

The 5' primer has the sequence 5' GCGGGATCCGCCATCATGTGGAAATGGATACTCAC 3' (SEQ ID NO. 7) and contains a BamHI restriction enzyme site (in bold) followed by 6 nucleotides resembling an efficient signal for the initiation of translation in eukaryotic cells (Kozak, M., J. Mol. Biol., 196:947-950 (1987). and just behind the first 17 nucleotides of the KGF-2 gene (the initiation codon for translation "ATG" is underlined).

The 3' primer has the sequence 5' GCGCGGTACCACAAACGTTGCCTTCCT 3' (SEQ ID No. 8) and contains the cleavage site for the restriction endonuclease Asp718 and 19 nucleotides complementary to the 3' non-translated sequence of the KGF-2 gene. The amplified sequences are isolated from a 1% agarose gel using a commercially available kit from Qiagen, Inc., Chatsworth, CA. The fragment is then digested with the endonucleases BamHI and Asp718 and then purified again on a 1% agarose gel. This fragment is designated F2.

The vector pA2 (modification of pVL941 vector, discussed below) is used for the expression of the KGF-2 protein using the baculovirus expression system (for review see: Summers, M.D. and Smith, G.E. 1987, A manual of methods for baculovirus vectors and insect cell culture procedures, Texas Agricultural Experimental Station Bulletin No. 1555). This expression vector contains the strong polyhedrin promoter of the Autographa californica nuclear polyhidrosis virus (AcMNPV) followed by the recognition sites for the restriction endonucleases BamHI and Asp718. The polyadenylation site of the simian virus (SV)40 is used for efficient polyadenylation. For an easy selection of recombinant viruses the beta-galactosidase gene from E.coli is inserted in the same orientation as the polyhedrin promoter followed by the polyadenylation signal of the polyhedrin gene. The polyhedrin sequences are flanked at both sides by viral sequences for the cell-mediated homologous recombination of co-transfected wild-type viral DNA. Many other baculovirus vectors could be used in place of pRG1 such as pAc373, pVL941 and pAcIM1 (Luckow, V.A. and Summers, M.D., Virology, 170:31-39).

The plasmid is digested with the restriction enzymes BamHI and Asp718. The DNA is then isolated from a 1% agarose gel using the commercially available kit (Qiagen, Inc., Chatsworth, CA). This vector DNA is designated V2.

Fragment F2 and the plasmid V2 are ligated with T4 DNA ligase. E.coli HB101 cells are then transformed and bacteria identified that contained the plasmid (pBacKGF-2) with the KGF-2 gene using PCR with both cloning oligonucleotides. The sequence of the cloned fragment is confirmed by DNA sequencing.

5 µg of the plasmid pBacKGF-2 is co-transfected with 1.0 µg of a commercially available linearized baculovirus ("BaculoGold™ baculovirus DNA", Pharmingen, San Diego, CA.) using the lipofection method (Felgner et al. Proc. Natl. Acad. Sci. USA, 84:7413-7417 (1987)).

1µg of BaculoGold™ virus DNA and 5 µg of the plasmid pBacKGF-2 are mixed in a sterile well of a microtiter plate containing 50 µl of serum free Grace's medium (Life Technologies Inc., Gaithersburg, MD). Afterwards 10 µl Lipofectin plus 90 µl Grace's medium are added, mixed and incubated for 15 minutes at room temperature. Then the transfection mixture is added drop-wise to the Sf9 insect cells (ATCC CRL 1711) seeded in a 35 mm tissue culture plate with 1 ml Grace's medium without serum. The plate is rocked back and forth to mix the newly added solution. The plate is then incubated for 5 hours at 27°C. After 5 hours the transfection solution is removed from the plate and 1 ml of Grace's insect medium supplemented with 10% fetal calf serum is added. The plate is put back into an incubator and cultivation continued at 27°C for four days.

After four days the supernatant is collected and a plaque assay performed similar as described by Summers and Smith (supra). As a modification an agarose gel with "Blue Gal" (Life Technologies Inc., Gaithersburg) is used which allows an easy isolation of blue stained plaques. (A detailed description of a "plaque assay" can also be found in the user's guide for insect cell culture and baculovirology distributed by Life Technologies Inc., Gaithersburg, page 9-10).

Four days after the serial dilution, the viruses are added to the cells and blue stained plaques are picked with the tip of an Eppendorf pipette. The agar containing the recombinant viruses is then resuspended in an Eppendorf tube containing 200 µl of Grace's medium. The agar is removed by a brief centrifugation and the supernatant containing the recombinant baculovirus is used to infect Sf9 cells seeded in 35 mm dishes. Four days later the supernatants of these culture dishes are harvested and then stored at 4°C.

Sf9 cells are grown in Grace's medium supplemented with 10% heat-inactivated FBS. The cells are infected with the recombinant baculovirus V-KGF-2 at a multiplicity of infection (MOI) of 2. Six hours later the medium is removed and replaced with SF900 II medium minus methionine and cysteine (Life Technologies Inc., Gaithersburg). 42 hours later 5 µCi of ³⁵S-methionine and 5 µCi ³⁵S cysteine (Amersham) are added. The cells are further incubated for 16 hours before they are harvested by centrifugation and the labelled proteins visualized by SDS-PAGE and autoradiography.

### Example 4

### Expression of Recombinant KGF-2 in COS cells

The expression of plasmid, KGF-2 HA is derived from a vector pcDNAI/Amp (Invitrogen) containing: 1) SV40 origin of replication, 2) ampicillin resistance gene, 3) E.coli replication origin, 4) CMV promoter followed by a polylinker region, a SV40 intron and polyadenylation site. A DNA fragment encoding the entire KGF-2 precursor and a HA tag fused in frame to its 3' end is cloned into the polylinker region of the vector, therefore, the recombinant protein expression is directed under the CMV promoter. The HA tag correspond to an epitope derived from the influenza hemagglutinin protein as previously described (I. Wilson, H. Niman, R. Heighten, A Cherenson, M. Connolly, and R. Lerner, 1984, Cell 37:767, (1984)). The infusion of HA tag to the target protein allows easy detection of the recombinant protein with an antibody that recognizes the HA epitope.

The plasmid construction strategy is described as follows:

The DNA sequence encoding KGF-2, ATCC # 75977, is constructed by PCR using two primers: the 5' primer 5' CCCAAGCTTATGTGGAAATGGATACTGACACATTGTGCC 3' (SEQ ID No. 9) contains a Hind III site followed by 30 nucleotides of KGF-2 coding sequence starting from the initiation codon; the 3' sequence 5' TGCTCTAGACTAAGCGTAGTCTGGGACGTCGTATGGGTATGAGTG TACCACCATTGGAAGAAAGTGAGG 3' (SEQ ID No. 10) contains complementary sequences to an XbaI site, translation stop codon, HA tag and the last 32 nucleotides of the KGF-2 coding sequence (not including the stop codon). Therefore, the PCR product contains a Hind III site, KGF-2 coding sequence followed by HA tag fused in frame, a translation termination stop codon next to the HA tag, and an XbaI site. The PCR amplified DNA fragment and the vector, pcDNAI/Amp, are digested with Hind III and Xba I restriction enzyme and ligated. The ligation mixture is transformed into B. coli strain XL1 Blue (Stratagene Cloning Systems, La Jolla, CA) the transformed culture is plated on ampicillin media plates and resistant colonies are selected. Plasmid DNA is isolated from transformants and examined by PCR and restriction analysis for the presence of the correct fragment. For expression of the recombinant KGF-2, COS cells are transfected with the expression vector by DEAE-DEXTRAN method (J. Sambrook, E. Fritsch, T. Maniatis, Molecular Cloning: A Laboratory Manual, Cold Spring Laboratory Press, (1989)). The expression of the KGF-2 HA protein is detected by radiolabelling and immunoprecipitation method (E. Harlow, D. Lane, Antibodies: A Laboratory Manual, Cold Spring Harbor Laboratory Press, (1988)). Cells are labelled for 8 hours with ³⁵S-cysteine two days post transfection. Culture media are then collected and cells are lysed with detergent (RIPA buffer (150 mM NaCl, 1% NP-40, 0.1% SDS, 1% NP-40, 0.5% DOC, 50mM Tris, pH 7.5) (Wilson, I. et al., Id. 37:767 (1984)). Both cell lysate and culture media are precipitated with a HA specific monoclonal antibody. Proteins precipitated are analyzed on 15% SDS-PAGE gels.

### Example 5

### Transcription and translation of recombinant KGF-2 in vitro :

A PCR product is derived from the cloned cDNA in the pA2 vector used for insect cell expression of KGF-2. The primers used for this PCR were:
5' ATTAACCCTCACTAAAGGGAGGCCATGTGGAAATGGATACTGACACATTGTGCC 3' (SEQ ID No. 11) and 5' CCCAAGCTTCCACAAACGTTGCCTTCCTCTATGAG 3' (SEQ ID No. 12).

The first primer contains the sequence of a T3 promoter 5' to the ATG initiation codon. The second primer is complimentary to the 3' end of the KGF-2 open reading frame, and encodes the reverse complement of a stop codon.

The resulting PCR product is purified using a commercially available kit from Qiagen. 0.5 µg of this DNA is used as a template for an in vitro transcription-translation reaction. The reaction is performed with a kit commercially available from Promega under the name of TNT. The assay is performed as described in the instructions for the kit, using radioactively labeled methionine as a substrate, with the exception that only 1/2 of the indicated volumes of reagents are used and that the reaction is allowed to proceed at 33°C for 1.5 hours.

Five µl of the reaction is electrophoretically separated on a denaturing 10 to 15% polyacrylamide gel. The gel is fixed for 30 minutes in a mixture of water:Methanol:Acetic acid at 6:3:1 volumes respectively. The gel is then dried under heat and vacuum and subsequently exposed to an X-ray film for 16 hours. The film is developed showing the presence of a radioactive protein band corresponding in size to the conceptually translated KGF-2, strongly suggesting that the cloned cDNA for KGF-2 contains an open reading frame that codes for a protein of the expected size.

Numerous modifications and variations of the present invention are possible in light of the above teachings and, therefore, within the scope of the appended claims, the invention may be practiced otherwise than as particularly described.

## Claims

1. A polynucleotide selected from the group consisting of
(a) polynucleotides comprising a nucleic acid sequence encoding amino acid residues -36 to +172 as set forth in SEQ ID NO: 2;
(b) polynucleotides comprising a nucleic acid sequence encoding amino acid residues +1 to +172 as set forth in SEQ ID NO:2;
(c) polynucleotides comprising a nucleic acid sequence encoding the full-length amino acid sequence encoded by the cDNA contained in ATCC 75977;
(d) polynucleotides comprising a nucleic acid sequence encoding the mature polypeptide encoded by the cDNA contained in ATCC 75977;
(e) polynucleotides comprising a nucleic acid sequence encoding a polypeptide fragment selected from the group consisting of:
(i) a polypeptide fragment of amino acid residues,-36 to +172 of SEQ ID NO:2;
(ii) a polypeptide fragment of amino acid residues +1 to +172 of SEQ ID NO:2;
(iii) a polypeptide fragment of the full-length polypeptide encoded by the cDNA contained in ATCC 75977; and
(iv) a polypeptide fragment of the mature polypeptide encoded by the cDNA contained in ATCC 75977;
wherein the polypeptide fragment stimulates proliferation of epithelial cells;
(f) polynucleotides comprising a first nucleic acid sequence which encodes a polypeptide which stimulates proliferation of epithelial cells and which is at least 95% identical to a second nucleotide sequence selected from the group consisting of:
(i) a nucleotide sequence encoding amino acids +1 to +172 of SEQ ID NO:2;
(ii) a nucleotide sequence encoding amino acids -36 to +172 of SEQ ID NO:2;
(iii) a nucleotide sequence encoding a mature polypeptide encoded by the cDNA contained in ATCC 75977;
(iv) a nucleotide sequence encoding the full-length polypeptide encoded by ATCC 75977;
(v) a nucleotide sequence encoding a polypeptide fragment of amino acid residues -36 to +172 of SEQ ID NO:2, wherein said polypeptide fragment stimulates proliferation of epithelial cells;
(vi) a nucleotide sequence encoding a polypeptide fragment of amino acid residues +1 to +172 of SEQ ID NO:2, wherein said polypeptide fragment stimulates proliferation of epithelial cells;
(vii) a nucleotide sequence encoding a polypeptide fragment of the full-length polypeptide encoded by the cDNA contained in ATCC 75977, wherein said polypeptide fragment stimulates proliferation of epithelial cells; and
(viii) a nucleotide sequence encoding a polypeptide fragment of the mature polypeptide encoded by the cDNA contained in ATCC 75977, wherein said polypeptide fragment stimulates proliferation of epithelial cells;
(g) the polynucleotide of (f), wherein said nucleotide sequence defined in any one of (i) to (viii) is selected from the group consisting of:
(i) nucleotide sequences comprising nucleotides 109 to 627 of SEQ ID NO:1;
(ii) nucleotide sequences comprising nucleotides 1 to 627 of SEQ ID NO:1;
(iii) nucleotide sequences comprising the cDNA encoding the mature polypeptide encoded by the cDNA of ATCC 75977; and
(iv) nucleotide sequences comprising the cDNA encoding the full-length polypeptide encoded by the cDNA of ATCC 75977;
(h) polynucleotides comprising a first nucleic acid sequence which encodes a polypeptide which stimulates proliferation of epithelial cells and which is at least 97% identical to a second nucleotide sequence selected from the group consisting of:
(i) a nucleotide sequence encoding amino acids +1 to +172 of SEQ ID NO:2;
(ii) a nucleotide sequence encoding amino acids -36 to +172 of SEQ ID NO:2;
(iii) a nucleotide sequence encoding a mature polypeptide encoded by the cDNA contained in ATCC 75977;
(iv) a nucleotide sequence encoding the full-length polypeptide encoded by ATCC 75977;
(v) a nucleotide sequence encoding a polypeptide fragment of amino acid residues -36 to +172 of SEQ ID NO:2, wherein said polypeptide fragment stimulates proliferation of epithelial cells;
(vi) a nucleotide sequence encoding a polypeptide fragment of amino acid residues +1 to +172 of SEQ ID NO:2, wherein said polypeptide fragment stimulates proliferation of epithelial cells;
(vii) a nucleotide sequence encoding a polypeptide fragment of the full-length polypeptide encoded by the cDNA contained in ATCC 75977, wherein said polypeptide fragment stimulates proliferation of epithelial cells; and
(viii) a nucleotide sequence encoding a polypeptide fragment of the mature polypeptide encoded by the cDNA contained in ATCC 75977, wherein said polypeptide fragment-stimulates proliferation of epithelial cells;
(i) polynucleotides of at least 30 contiguous nucleotides of SEQ ID NO: 1;
(j) polynucleotides of at least 30 contiguous nucleotides of the cDNA contained in ATCC 75977;
(k) polynucleotides of 50 contiguous nucleotides of SEQ ID NO. 1; and
(l) polynucleotides of 50 contiguous nucleotides of the cDNA contained in ATCC 75977;
(m) polynucleotides of a greater number than 50 contiguous nucleotides of SEQ ID NO:1; and
(n) polynucleotides of a greater number than 50 contiguous nucleotides of the cDNA contained in ATCC 75977;
or the complementary strand of such a polynucleotide.

2. The polynucleotide of claim 1, wherein the epithelial cells are keratinocytes.

3. The polynucleotide of claim 1 or 2, wherein said polynucleotide does not encode an initial methionine amino acid residue.

4. The polyriucleotide of claim 1 or 2, wherein said polynucleotide encodes a N-terminal methionine.

5. The polynucleotide of any one of claims 1 to 4, wherein said polynucleotide is DNA or RNA.

6. The polynucleotide of claim 5 which is cDNA or genomic DNA.

7. The polynucleotide of any one of claims 1 to 6, wherein said polynucleotide is double stranded or single stranded.

8. The polynucleotide of any one of claims 1 to 7, which is fused to a heterologous polynucleotide.

9. The polynucleotide of claim 8, wherein the heterologous polynucleotide encodes a heterologous polypeptide.

10. The polynucleotide of claim 9, wherein said heterologous polypeptide is fused to a polypeptide encoded by said polynucleotide.

11. A vector comprising the polynucleotide of any one of claims 1 to 10.

12. The vector of claim 11, wherein the polynucleotide is operably associated with a regulatory control sequence.

13. A method of producing a host cell comprising genetically engineering cells with the vector of claim 11 or 12.

14. A host cell produced by the method of claim 13.

15. A host cell comprising the vector of claim 11 or 12.

16. A genetically engineered host cell comprising the polynucleotide of any one of claims 1 to 10 operably associated with a heterologous regulatory control sequence.

17. The host cell of any one of claims 14 to 16, wherein said host cell is a prokaryotic cell, eukaryotic cell, mammalian cell, COS cell, CHO cell, or E. coli cell.

18. A method of producing a polypeptide, comprising expressing from the host cell of any one of claims 14 to 17, the polypeptide encoded by said polynucleotide and recovering said polypeptide.

19. A method of producing a polypeptide, comprising expressing a polypeptide encoded by the polynucleotide of any one of claims 1 to 10 and recovering said polypeptide.

20. A polypeptide comprising an amino acid sequence encoded by a polynucleotide of any one of claims 1 to 10.

21. The polypeptide of claim 20, wherein said polypeptide is chemically synthesized, or can be obtained by the host cell of any one of claims 14 to 17 or which is obtainable by the method of claim 18 or 19.

22. The polypeptide of claim 20 or 21, wherein the polypeptide is labeled, modified, or pegylated.

23. The polypeptide of any one of claims 20 to 22 which is fused to a heterologous polypeptide.

24. The polypeptide of any one of claims 20 to 23, wherein said polypeptide lacks an N-terminal methionine.

25. The polypeptide of any one of claims 20 to 23, wherein said polypeptide has an N-terminal methionine.

26. An antibody specifically recognizing the polypeptide of any one of claims 20 to 25.

27. The antibody of claim 26 which is polyclonal, monoclonal, chimeric, single chain, human antibody, humanized antibody, or Fab fragment.

28. The antibody of claim 26 or 27, wherein said antibody is immobilized.

29. A pharmaceutical composition comprising the polynucleotide of any one of claims 1 to 10, the polypeptide of any one of claims 20 to 25 or the antibody of any one of claims 26 to 28.

30. Use of the polynucleotide of any one of claims 1 to 10 or the polypeptide of any one of claims 20 to 25 for the preparation of a pharmaceutical composition for promoting proliferation of epithelial cells.

31. Use of the polynucleotide of any one of claims 1 to 10 or the polypeptide of any one of claims 20 to 25 for the preparation of a pharmaceutical composition for stimulating keratinocytes.

32. Use of the polynucleotide of any one of claims 1 to 10 or the polypeptide of any one of claims 20 to 25 for the preparation of a pharmaceutical composition for the stimulation of the healing of wounds, hair follicle production or the healing of dermal wounds.

33. Use of the polynucleotide of any one of claims 1 to 10 or the polypeptide of any one of claims 20 to 25 for the preparation of a pharmaceutical composition for reducing scarring.

34. The use of any one of claims 30 to 33, wherein the pharmaceutical composition is suitable for the user in a human.

## Patentansprüche

1. Polynucleotid, ausgewählt aus der Gruppe bestehend aus
(a) Polynucleotiden, umfassend eine Nucleinsäuresequenz, welche die wie in SEQ ID NO:2 dargestellten Aminosäurereste -36 bis +172 codiert;
(b) Polynucleotiden, umfassend eine Nucleinsäuresequenz, welche die wie in SEQ ID NO:2 dargestellten Aminosäurereste +1 bis +172 codiert;
(c) Polynucleotiden, umfassend eine Nucleinsäuresequenz, welche die Voll-Länge Aminosäuresequenz codiert, die von der in ATCC 75977 enthaltenen cDNA codiert wird;
(d) Polynucleotiden, umfassend eine Nucleinsäuresequenz, welche das reife Polypeptid codiert, das von der in ATCC 75977 enthaltenen cDNA codiert wird;
(e) Polynucleotiden, umfassend eine Nucleinsäuresequenz, welche ein Polypeptidfragment codiert, ausgewählt aus der Gruppe bestehend aus:
(i) einem Polypeptidfragment der Aminosäurereste -36 bis +172 von SEQ ID NO:2;
(ii) einem Polypeptidfragment der Aminosäurereste +1 bis +172 von SEQ ID NO:2;
(iii) einem Polypeptidfragment des Voll-Länge Polypeptids, das von der in ATCC 75977 enthaltenen cDNA codiert wird; und
(iv) einem Polypeptidfragment des reifen Polypeptids, das von der in ATCC 75977 enthaltenen cDNA codiert wird;
wobei das Polypeptidfragment Proliferation von Epithelzellen stimuliert;
(f) Polynucleotiden, umfassend eine erste Nucleinsäuresequenz, welche ein Polypeptid codiert, das Proliferation von Epithelzellen stimuliert und welche zu mindestens 95% identisch ist mit einer zweiten Nucleinsäuresequenz, ausgewählt aus der Gruppe bestehend aus:
(i) einer Nucleotidsequenz, welche die Aminosäuren +1 bis +172 von SEQ ID NO:2 codiert;
(ii) einer Nucleotidsequenz, welche die Aminosäuren -36 bis +172 von SEQ ID NO:2 codiert,
(iii) einer Nucleotidsequenz, welche ein reifes Polypeptid codiert, das von der in ATCC 75977 enthaltenen cDNA codiert wird;
(iv) einer Nucleotidsequenz, welche das Voll-Länge Polypeptid codiert, das von ATCC 75977 codiert wird;
(v) einer Nucleotidsequenz, welche ein Polypeptidfragment der Aminosäurereste -36 bis +172 von SEQ ID NO:2 codiert, wobei das Polypeptidfragment Proliferation von Epithelzellen stimuliert;
(vi) einer Nucleotidsequenz, welche ein Polypeptidfragment der Aminosäurereste +1 bis +172 von SEQ ID NO:2 codiert, wobei das Polypeptidfragment Proliferation von Epithelzellen stimuliert;
(vii) einer Nucleotidsequenz, welche ein Polypeptidfragment des Voll-Länge Polypeptids codiert, welches von der in ATCC 75977 enthaltenen cDNA codiert wird, wobei das Polypeptidfragment Proliferation von Epithelzellen stimuliert; und
(viii) einer Nucleotidsequenz, welche ein Polypeptidfragment des reifen Polypeptids codiert, welches von der in ATCC 75977 enthaltenen cDNA codiert wird, wobei das Polypeptidfragment Proliferation von Epithelzellen stimuliert;
(g) dem Polynucleotid von (f), wobei die Nucleotidsequenz, die in einem der Teile (i) bis (viii) definiert ist, ausgewählt ist aus der Gruppe bestehend aus
(i) Nucleotidsequenzen, umfassend die Nucleotide 109 bis 627 von SEQ ID NO:1;
(ii) Nucleotidsequenzen, umfassend die Nucleotide 1 bis 627 von SEQ ID NO:1;
(iii) Nucleotidsequenzen, umfassend die cDNA, welche das reife Polypeptid codiert, das von der in ATCC 75977 enthaltenen cDNA codiert wird; und
(iv) Nucleotidsequenzen, umfassend die cDNA, welche das Voll-Länge Polypeptid codiert, das von der in ATCC 75977 enthaltenen cDNA codiert wird;
(h) Polynucleotiden, umfassend eine erste Nucleinsäuresequenz, welche ein Polypeptid codiert, das Proliferation von Epithelzellen stimuliert und welche zu mindestens 95% identisch ist mit einer zweiten Nucleinsäuresequenz, ausgewählt aus der Gruppe bestehend aus:
(i) einer Nucleotidsequenz, welche die Aminosäuren +1 bis +172 von SEQ ID NO:2 codiert;
(ii) einer Nucleotidsequenz, welche die Aminosäuren -36 bis +172 von SEQ ID NO:2 codiert,
(iii) einer Nucleotidsequenz, welche ein reifes Polypeptid codiert, das von der in ATCC 75977 enthaltenen cDNA codiert wird;
(iv) einer Nucleotidsequenz, welche das Voll-Länge Polypeptid codiert, das von ATCC 75977 codiert wird;
(v) einer Nucleotidsequenz, welche ein Polypeptidfragment der Aminosäurereste -36 bis +172 von SEQ ID NO:2 codiert, wobei das Polypeptidfragment Proliferation von Epithelzellen stimuliert;
(vi) einer Nucleotidsequenz, welche ein Polypeptidfragment der Aminosäurereste +1 bis +172 von SEQ ID NO:2 codiert, wobei das Polypeptidfragment Proliferation von Epithelzellen stimuliert;
(vii) einer Nucleotidsequenz, welche ein Polypeptidfragment des Voll-Länge Polypeptids codiert, welches von der in ATCC 75977 enthaltenen cDNA codiert wird, wobei das Polypeptidfragment Proliferation von Epithelzellen stimuliert; und
(viii) einer Nucleotidsequenz, welche ein Polypeptidfragment des reifen Polypeptids codiert, welches von der in ATCC 75977 enthaltenen cDNA codiert wird, wobei das Polypeptidfragment Proliferation von Epithelzellen stimuliert;
(i) Polynucleotiden von mindestens 30 zusammenhängenden Nucleotiden von SEQ ID NO:1;
(j) Polynucleotiden von mindestens 30 zusammenhängenden Nucleotiden der in ATCC 75977 enthaltenen cDNA;
(k) Polynucleotiden von 50 zusammenhängenden Nucleotiden von SEQ ID NO:1; und
(l) Polynucleotiden von 50 zusammenhängenden Nucleotides der in ATCC 75977 enthaltenen cDNA;
(m) Polynucleotiden einer größeren Anzahl als 50 zusammenhängenden Nucleotiden von SEQ ID NO:1; und
(n) Polynucleotiden einer größeren Anzahl als 50 zusammenhängenden Nucleotiden der in ATCC 75977 enthaltenen cDNA;
oder der komplementäre Strang eines solchen Polynucleotids.

2. Polynucleotid nach Anspruch 1, wobei die Epithelzellen Keratinozyten sind.

3. Polynucleotid nach Anspruch 1 oder 2, wobei das Polynucleotid keinen Methionin-Aminosäurerest am Anfang codiert.

4. Polynucleotid nach Anspruch 1 oder 2, wobei das Polynucleotid ein N-terminales Methionin codiert.

5. Polynucleotid nach einem der Ansprüche 1 bis 4, wobei das Polynucleotid DNA oder RNA ist.

6. Polynucleotid nach Anspruch 5, das cDNA oder genomische DNA ist.

7. Polynucleotid nach einem der Ansprüche 1 bis 6, wobei das Polynucleotid doppelsträngig oder einzelsträngig ist.

8. Polynucleotid nach einem der Ansprüche 1 bis 7, das mit einem heterologen Polynucleotid fusioniert ist.

9. Polynucleotid nach Anspruch 8, wobei das heterologe Polynucleotid ein heterologes Polypeptid codiert.

10. Polynucleotid nach Anspruch 9, wobei das heterologe Polypeptid mit einem Polypeptid fusioniert ist, welches von dem Polynucleotid codiert wird.

11. Vektor, umfassend das Polynucleotid nach einem der Ansprüche 1 bis 10.

12. Vektor nach Anspruch 11, wobei das Polynucleotid funktionell mit einer regulatorischen Kontrollsequenz verbunden ist.

13. Verfahren zur Herstellung einer Wirtszelle, umfassend genetisches Verändern von Zellen mit dem Vektor nach Anspruch 11 oder 12.

14. Wirtszelle, hergestellt nach dem Verfahren nach Anspruch 13.

15. Wirtszelle, umfassend den Vektor nach Anspruch 11 oder 12.

16. Genetisch veränderte Wirtszelle, umfassend das Polynucleotid nach einem der Ansprüche 1 bis 10, das funktionell mit einer heterologen regulatorischen Koritrollsequenz verbunden ist.

17. Wirtszelle nach einem der Ansprüche 14 bis 16, wobei die Wirtszelle eine prokaryotische Zelle, eukaryotische Zelle, Säugerzelle, COS-Zelle, CHO-Zelle oder E. coli-Zelle ist.

18. Verfahren zur Herstellung eines Polypeptids, umfassend Exprimieren des Polypeptids von der Wirtszelle nach einem der Ansprüche 14 bis 16, das von dem Polynucleotid codiert wird, und Gewinnen des Polypeptids.

19. Verfahren zur Herstellung eines Polypeptids, umfassend Exprimieren eines Polypeptids, das von dem Polynucleotid nach einem der Ansprüche 1 bis 10 codiert wird, und Gewinnen des Polypeptids.

20. Polypeptid, umfassend eine Aminosäuresequenz, die von einem Polynucleotid nach einem der Ansprüche 1 bis 10 codiert wird.

21. Polypeptid nach Anspruch 20, wobei das Polypeptid chemisch synthetisiert ist, oder von der Wirtszelle nach einem der Ansprüche 14 bis 17 erhalten werden kann oder das erhältlich ist durch das Verfahren nach Anspruch 18 oder 19.

22. Polypeptid nach Anspruch 20 oder 21, wobei das Polypeptid markiert, modifiziert oder pegyliert ist.

23. Polypeptid nach einem der Ansprüche 20 bis 22, das mit einem heterologen Polypeptid fusioniert ist.

24. Polypeptid nach einem der Ansprüche 20 bis 23, wobei dem Polypeptid ein N-terminales Methionin fehlt.

25. Polypeptid nach einem der Ansprüche 20 bis 23, wobei das Polypeptid ein N-terminales Methionin besitzt.

26. Antikörper, der spezifisch das Polypeptid nach einem der Ansprüche 20 bis 25 erkennt.

27. Antikörper nach Anspruch 26, der polyclonal, monoclonal, chimär, einzelkettig, ein menschlicher Antikörper, humanisierter Antikörper oder ein Fab-Fragment ist.

28. Antikörper nach Anspruch 26 oder 27, wobei der Antikörper immobilisiert ist.

29. Arzneimittel, umfassend das Polynucleotid nach einem der Ansprüche 1 bis 10, das Polypeptid nach einem der Ansprüche 20 bis 25 oder den Antikörper nach einem der Ansprüche 26 bis 28.

30. Verwendung des Polynucleotids nach einem der Ansprüche 1 bis 10 oder des Polypeptids nach einem der Ansprüche 20 bis 25 zur Herstellung eines Arzneimittels zum Fördern der Proliferation von Epithelzellen.

31. Verwendung des Polynucleotids nach einem der Ansprüche 1 bis 10 oder des Polypeptids nach einem der Ansprüche 20 bis 25 zur Herstellung eines Arzneimittels zum Stimulieren von Keratinozyten.

32. Verwendung des Polynucleotids nach einem der Ansprüche 1 bis 10 oder des Polypeptids nach einem der Ansprüche 20 bis 25 zur Herstellung eines Arzneimittels für die Stimulierung des Heilens von Wunden, die Produktion von Haarfollikeln oder das Heilen von Hautwunden.

33. Verwendung des Polynucleotids nach einem der Ansprüche 1 bis 10 oder des Polypeptids nach einem der Ansprüche 20 bis 25 zur Herstellung eines Arzneimittels zum Reduzieren von Narbenbildung.

34. Verwendung nach einem der Ansprüche 30 bis 33, wobei das Arzneimittel für die Verwendung in einem Menschen geeignet ist.

## Revendications

1. Polynucléotide sélectionné parmi le groupe constitué de
(a) polynucléotides comportant une séquence d'acide nucléique codant pour les résidus d'acide aminé -36 à +172 comme établi dans la SEQ ID N° 2,
(b) polynucléotides comportant une séquence d'acide nucléique codant pour les résidus d'acide aminé +1 à +172 comme établi dans la SEQ ID N° 2,
(c) polynucléotides comportant une séquence d'acide nucléique codant pour la séquence d'acides aminés de longueur entière codée par l'ADNc contenu dans ATCC 75977,
(d) polynucléotides comportant une séquence d'acide nucléique codant pour le polypeptide mature codé par l'ADNc contenu dans ATCC 75977,
(e) polynucléotides comportant une séquence d'acide nucléique codant pour un fragment polypeptidique sélectionné parmi le groupe constitué de :
(i) un fragment polypeptidique de résidus d'acide aminé -36 à +172 de la SEQ ID N° 2,
(ii) un fragment polypeptidique de résidus d'acide aminé +1 à +172 de la SEQ ID N° 2,
(iii) un fragment polypeptidique du polypeptide de longueur entière codé par l'ADNc contenu dans ATCC 75977, et
(iv) un fragment polypeptidique du polypeptide mature codé par l'ADNc contenu dans ATCC 75977,
dans lequel le fragment polypeptidique stimule une prolifération de cellules épithéliales,
(f) polynucléotides comportant une première séquence d'acide nucléique qui code pour une polypeptide qui stimule la prolifération de cellules épithéliales et qui est au moins identique à 95 % avec une seconde séquence nucléotidique sélectionnée parmi le groupe constitué de :
(i) une séquence nucléotidique codant pour les acides aminés +1 à +172 de la SEQ ID N° 2,
(ii) une séquence nucléotidique codant pour les acides aminés -36 à +172 de la SEQ ID N° 2,
(iii) une séquence nucléotidique codant pour un polypeptide mature codé par l'ADNc contenu dans ATCC 75977,
(iv) une séquence nucléotidique codant pour le polypeptide de longueur entière codé par ATCC 75977,
(v) une séquence nucléotidique codant pour un fragment polypeptidique des résidus d'acide aminé -36 à +172 de la SEQ ID N° 2, ledit fragment polypeptidique stimulant une prolifération de cellules épithéliales,
(vi) une séquence nucléotidique codant pour un fragment polypeptidique des résidus d'acide aminé +1 à +172 de la SEQ ID N° 2, ledit fragment polypeptidique stimulant une prolifération de cellules épithéliales,
(vii) une séquence nucléotidique codant pour un fragment polypeptidique du polypeptide de longueur entière codé par l'ADNc contenu dans ATCC 75977, ledit fragment polypeptidique stimulant une prolifération de cellules épithéliales, et
(viii) une séquence nucléotidique codant pour un fragment polypeptidique du polypeptide mature codé par l'ADNc contenu dans ATCC 75977, ledit fragment polypeptidique stimulant une prolifération de cellules épithéliales,
(g) polynucléotide de (f), dans lequel la séquence nucléotidique définie selon l'un quelconque des points (i) à (viii) est sélectionnée parmi le groupe constitué de :
(i) séquences nucléotidiques comportant les nucléotides 109 à 627 de la SEQ ID N° 1,
(ii) séquences nucléotidiques comportant les nucléotides 1 à 627 de la SEQ ID N° 1,
(iii) séquences nucléotidiques comportant l'ADNc codant pour le polypeptide mature codé par l'ADNc de ATCC 75977, et
(iv) séquences nucléotidiques comportant l'ADNc codant pour le polypeptide de longueur entière codé par l'ADNc de ATCC 75977,
(h) polynucléotides comportant une première séquence d'acide nucléique qui code pour un polypeptide qui stimule la prolifération de cellules épithéliales et qui est au moins identique à 97 % avec une seconde séquence nucléotidique sélectionnée parmi le groupe constitué de
(i) une séquence nucléotidique codant pour les acides aminés +1 à +172 de la SEQ ID N° 2,
(ii) une séquence nucléotidique codant pour les acides aminés -36 à +172 de la SEQ ID N° 2,
(iii) une séquence nucléotidique codant pour un polypeptide mature codé par l'ADNc contenu dans ATCC 75977,
(iv) une séquence nucléotidique codant pour le polypeptide de longueur entière codé par ATCC 75977,
(v) une séquence nucléotidique codant pour un fragment polypeptidique des résidus d'acide aminé -36 à +172 de la SEQ ID N° 2, ledit fragment polypeptidique stimulant une prolifération de cellules épithéliales,
(vi) une séquence nucléotidique codant pour un fragment polypeptidique des résidus d'acide aminé +1 à +172 de la SEQ ID N° 2, ledit fragment polypeptidique stimulant une prolifération de cellules épithéliales,
(vii) une séquence nucléotidique codant pour un fragment polypeptidique du polypeptide de longueur entière codé par l'ADNc contenu dans ATCC 75977, ledit fragment polypeptidique stimulant la prolifération de cellules épithéliales, et
(viii) une séquence nucléotidique codant pour un fragment polypeptidique du polypeptide mature codé par l'ADNc contenu dans ATCC 75977, ledit fragment polypeptidique stimulant une prolifération de cellules épithéliales,
(i) polynucléotides d'au moins 30 nucléotides contigus de la SEQ ID N° 1,
(j) polynucléotides d'au moins 30 nucléotides contigus de l'ADNc contenu dans ATCC 75977,
(k) polynucléotides de 50 nucléotides contigus de la SEQ ID N° 1, et
(l) polynucléotides de 50 nucléotides contigus de l'ADNc contenu dans ATCC 75977,
(m) polynucléotides ayant un nombre supérieur à 50 nucléotides contigus de la SEQ ID N° 1, et
(n) polynucléotides ayant un nombre supérieur à 50 nucléotides contigus de l'ADNc contenu dans ATCC 75977,
ou le brin complémentaire d'un tel polynucléotide

2. Polynucléotide selon la revendication 1, dans lequel les cellules épithéliales sont des kératinocytes.

3. Polynucléotide selon la revendication 1 ou 2, dans lequel ledit polynucléotide ne code pas pour un résidu d'acide aminé méthionine initial.

4. Polynucléotide selon la revendication 1 ou 2, dans lequel ledit polynucléotide code pour une méthionine N-terminale.

5. Polynucléotide selon l'une quelconque des revendications 1 à 4, dans lequel ledit polynucléotide est un ADN ou un ARN.

6. Polynucléotide selon la revendication 5, qui est un ADNC ou un ADN génomique.

7. Polynucléotide selon l'une quelconque des revendications 1 à 6, dans lequel ledit polynucléotide est de nature double-brin ou simple-brin.

8. Polynucléotide selon l'une quelconque des revendications 1 à 7, qui est fusionné à un polynucléotide hétérologue.

9. Polynucléotide selon la revendication 8, dans lequel le polynucléotide hétérologue code pour un polypeptide hétérologue.

10. Polynucléotide selon la revendication 9, dans lequel ledit polypeptide hétérologue est fusionné à un polypeptide codé par ledit polynucléotide.

11. Vecteur comportant le polynucléotide selon l'une quelconque des revendications 1 à 10.

12. Vecteur selon la revendication 11, dans lequel le polynucléotide est associé de manière opérationnelle à une séquence de contrôle de régulation.

13. Procédé de production d'une cellule hôte comportant la manipulation génétique des cellules à l'aide du vecteur selon la revendication 11 ou 12.

14. Cellule hôte produite par le procédé selon la revendication 13.

15. Cellule hôte comportant le vecteur selon la revendication 11 ou 12.

16. Cellule hôte manipulée génétiquement comportant le polynucléotide selon l'une quelconque des revendications 1 à 10, associé de manière opérationnelle à une séquence de contrôle de régulation hétérologue.

17. Cellule hôte selon l'une quelconque des revendications 14 à 16, dans laquelle ladite cellule hôte est une cellule procaryote, une cellule eucaryote, une cellule mammifère, une cellule COS, une cellule CHO, ou une cellule de E. coli.

18. Procédé de production d'un polypeptide, comportant l'expression, à partir de la cellule hôte selon l'une quelconque des revendications 14 à 17, du polypeptide codé par ledit polynucléotide et la récupération dudit polypeptide.

19. Procédé de production d'un polypeptide, comportant l'expression d'un polypeptide codé par le polynucléotide selon l'une quelconque des revendications 1 à 10 et la récupération dudit polypeptide.

20. Polypeptide comportant une séquence d'acides aminés codée par un polynucléotide selon l'une quelconque des revendications 1 à 10.

21. Polypeptide selon la revendication 20, dans lequel ledit polypeptide est synthétisé chimiquement, ou peut être obtenu par la cellule hôte selon l'une quelconque des revendications 14 à 17 ou qui peut être obtenu par le procédé selon la revendication 18 ou 19.

22. Polypeptide selon la revendication 20 ou 21, dans lequel le polypeptide est marqué, modifié, ou pégylé.

23. Polypeptide selon l'une quelconque des revendications 20 à 22 qui est fusionné à un polypeptide hétérologue.

24. Polypeptide selon l'une quelconque des revendications 20 à 23, dans lequel ledit polypeptide ne présente pas une méthionine N-terminale.

25. Polypeptide selon l'une quelconque des revendications 20 à 23, dans lequel ledit polypeptide présente une méthionine N-terminale.

26. Anticorps reconnaissant de manière spécifique le polypeptide selon l'une quelconque des revendications 20 à 25.

27. Anticorps selon la revendication 26, qui est un anticorps polyclonal, monoclonal, chimère, à chaîne unique, humain, un anticorps humanisé ou un fragment Fab.

28. Anticorps selon la revendication 26 ou 27, dans lequel ledit anticorps est immobilisé.

29. Composition pharmaceutique comportant le polynucléotide selon l'une quelconque des revendications 1 à 10, le polypeptide selon l'une quelconque des revendications 20 à 25 ou l'anticorps selon l'une quelconque des revendications 26 à 28.

30. Utilisation du polynucléotide selon l'une quelconque des revendications 1 à 10 ou du polypeptide selon l'une quelconque des revendications 20 à 25 pour la préparation d'une composition pharmaceutique destinée à favoriser la prolifération de cellules épithéliales.

31. Utilisation du polynucléotide selon l'une quelconque des revendications 1 à 10 ou du polypeptide selon l'une quelconque des revendications 20 à 25 pour la préparation d'une composition pharmaceutique destinée à stimuler des kératinocytes.

32. Utilisation du polynucléotide selon l'une quelconque des revendications 1 à 10 ou du polypeptide selon l'une quelconque des revendications 20 à 25 pour la préparation d'une composition pharmaceutique destinée à stimuler la guérison de plaies, la production de follicules pileux ou la guérison de plaies dermiques.

33. Utilisation du polynucléotide selon l'une quelconque des revendications 1 à 10 ou du polypeptide selon l'une quelconque des revendications 20 à 25 pour la préparation d'une composition pharmaceutique destinée à réduire la cicatrisation.

34. Utilisation selon l'une quelconque des revendications 30 à 33, dans laquelle la composition pharmaceutique est adaptée pour être utilisée avec un être humain.


### SEQUENCE LISTING

(1) GENERAL INFORMATION:
   (i) APPLICANT: GRUBER, ET AL.
   (ii) TITLE OF INVENTION: Keratinocyte Growth Factor-2
   (iii) NUMBER OF SEQUENCES: 12
   (iv) CORRESPONDENCE ADDRESS:
      (A) ADDRESSEE: CARELLA, BYRNE, BAIN, GILFILLAN, CECCHI, STEWART & OLSTEIN
      (B) STREET: 6 BECKER FARM ROAD
      (C) CITY: ROSELAND
      (D) STATE: NEW JERSEY
      (E) COUNTRY: USA
      (F) ZIP: 07068
   (v) COMPUTER READABLE FORM:
      (A) MEDIUM TYPE: 3.5 INCH DISKETTE
      (B) COMPUTER: IBM PS/2
      (C) OPERATING SYSTEM: MS-DOS
      (D) SOFTWARE: WORD PERFECT 5.1
   (vi) CURRENT APPLICATION DATA:
      (A) APPLICATION NUMBER:
      (B) FILING DATE: Concurrently
      (C) CLASSIFICATION:
   (vii) PRIOR APPLICATION DATA
      (A) APPLICATION NUMBER:
      (B) FILING DATE:
   (viii) ATTORNEY/AGEMT INFORMATION:
      (A) NAME: FERRARO, GREGORY D.
      (B) REGISTRATION NUMBER: 36,134
      (C) REFERENCE/DOCKET NUMBER: 325800-261
   (ix) TELECOMMUNICATION INFORMATION:
      (A) TELEPHONE: 201-994-1700
      (B) TELEFAX: 201-994-1744
(2) INFORMATION FOR SEQ ID NO:1:
   (i) SEQUENCE CHARACTERISTICS
      (A) LENGTH: 627 BASE PAIRS
      (B) TYPE: NUCLEIC ACID
      (C) STRANDEDNESS: SINGLE
      (D) TOPOLOGY: LINEAR
   (ii) MOLECULE TYPE: cDNA
   (xi) SEQUENCE DESCRIPTION: SEQ ID NO:1:
   ATGTGGAAAT GGATACTGAC ACATTGTGCC TCAGCCTTTC CCCACCTGCC CGGCTGCTGC   60
   TGCTGCTGCT TTTTGTTGCT GTTCTTGGTG TCTTCCGTCC CTGTCACCTG CCAAGCCCTT  120
   GGTCAGGACA TGGTGTCACC AGAGGCCACC AACTCTTCTT CCTCCTCCTT CTCCTCTCCT  180
   TCCAGCGCGG GAAGGCATGT GCGGAGCTAC AATCACCTTC AAGGAGATGT CCGCTGGAGA  240
   AAGCTATTCT CTTTCACCAA GTACTTTCTC AAGATTGAGA AGAACGGGAA GGTCAGCGGG  300
   ACCAAGAAGG AGAACTGCCC GTACAGCATC CTGGAGATAA CATCAGTAGA AATCGGAGTT  360
   GTTGCCGTCA AAGCCATTAA CAGCAACTGT TACTTAGCCA TGAACAAGAA GGGGAAACTC  420
   TATGGCTCAA AAGAATTTAA CAATGACTGT AAGCTGAAGG AGAGGATAGA GGAAAATGGA  480
   TACAATACCT ATGCATCATT TAACTGGCAG CATAATGGGA GGCAAATGTA TGTGGCATTG  540
   AATGGAAAAG GAGCTCCAAG GAGAGGACAG AAAACACGAA GGAAAAACAC CTCTGCTCAC  600
   TTTCTTCCAA TGGTGGTACA CTCATAG                                      627
(2) INFORMATION FOR SEQ ID NO:2:
   (i) SEQUENCE CHARACTERISTICS
      (A) LENGTH: 208 AMINO ACIDS
      (B) TYPE: AMINO ACID
      (C) STRANDEDNESS:
      (D) TOPOLOGY: LINEAR
   (ii) MOLECULE TYPE: PROTEIN
   (xi) SEQUENCE DESCRIPTION: SEQ ID NO:2:
(2) INFORMATION FOR SEQ ID NO:3:
   (i) SEQUENCE CHARACTERISTICS
      (A) LENGTH: 36 BASE PAIRS
      (B) TYPE: NUCLEIC ACID
      (C) STRANDEDNESS: SINGLE
      (D) TOPOLOGY: LINEAR
   (ii) MOLECULE TYPE: Oligonucleotide
   (xi) SEQUENCE DESCRIPTION: SEQ ID NO: 3:
   CCCCACATGT GGAAATGGAT ACTGACACAT TGTGCC 36
(2) INFORMATION FOR SEQ ID NO:4:
   (i) SEQUENCE CHARACTERISTICS
      (A) LENGTH: 35 BASE PAIRS
      (B) TYPE: NUCLEIC ACID
      (C) STRANDEDNESS: SINGLE
      (D) TOPOLOGY: LINEAR
   (ii) MOLECULE TYPE: Oligonucleotide
   (xi) SEQUENCE DESCRIPTION: SEQ ID NO:4:
   CCCAAGCTTC CACAAACGTT GCCTTCCTCT ATGAG 35
(2) INFORMATION FOR SEQ ID NO:5:
   (i) SEQUENCE CHARACTERISTICS
      (A) LENGTH: 36 BASE PAIRS
      (B) TYPE: NUCLEIC ACID
      (C) STRANDEDNESS: SINGLE
      (D) TOPOLOGY: LINEAR
   (ii) MOLECULE TYPE: Oligonucleotide
   (xi) SEQUENCE DESCRIPTION: SEQ ID NO:5:
   CATGCCATGG CGTGCCAAGC CCTTGGTCAG GACATG 36
(2) INFORMATION FOR SEQ ID NO:6:
   (i) SEQUENCE CHARACTERISTICS
      (A) LENGTH: 35 BASE PAIRS
      (B) TYPE: NUCLEIC ACID
      (C) STRANDEDNESS: SINGLE
      (D) TOPOLOGY: LINEAR
   (ii) MOLECULE TYPE: Oligonucleotide
   (xi) SEQUENCE DESCRIPTION: SEQ ID NO:6:
   CCCAAGCTTC CACAAACGTT GCCTTCCTCT ATGAG 35
(2) INFORMATION FOR SEQ ID NO: 7:
   (i) SEQUENCE CHARACTERISTICS
      (A) LENGTH: 35 BASE PAIRS
      (B) TYPE: NUCLEIC ACID
      (C) STRANDEDNESS: SINGLE
      (D) TOPOLOGY: LINEAR
   (ii) MOLECULE TYPE: Oligonucleotide
   (xi) SEQUENCE DESCRIPTION: SEQ ID NO:7:
   GCGGGATCCG CCATCATGTG GAAATGGATA CTCAC 35
(2) INFORMATION FOR SEQ ID NO: 8:
   (i) SEQUENCE CHARACTERISTICS
      (A) LENGTH: 26 BASE PAIRS
      (B) TYPE: NUCLEIC ACID
      (C) STRANDEDNESS: SINGLE
      (D) TOPOLOGY: LINEAR
   (ii) MOLECULE TYPE: Oligonucleotide
   (xi) SEQUENCE DESCRIPTION: SEQ ID NO: 8:
   CGCGGTACCA CAAACGTTGC CTTCCT 26
(2) INFORMATION FOR SEQ ID NO:9:
   (i) SEQUENCE CHARACTERISTICS
      (A) LENGTH: 39 BASE PAIRS
      (B) TYPE: NUCLEIC ACID
      (C) STRANDEDNESS: SINGLE
      (D) TOPOLOGY: LINEAR
   (ii) MOLECULE TYPE: Oligonucleotide
   (xi) SEQUENCE DESCRIPTION: SEQ ID NO:9:
   CCCAAGCTTA TGTGGAAATG GATACTGACA CATTGTGCC 39
(2) INFORMATION FOR SEQ ID NO:10:
   (i) SEQUENCE CHARACTERISTICS
      (A) LENGTH: 69 BASE PAIRS
      (B) TYPE: NUCLEIC ACID
      (C) STRANDEDNESS: SINGLE
      (D) TOPOLOGY: LINEAR
   (ii) MOLECULE TYPE: Oligonucleotide
   (xi) SEQUENCE DESCRIPTION: SEQ ID NO:10:
   TGCTCTAGAC TAAGCGTAGT CTGGGACGTC GTATGGGTAT GAGTGTACCA CCATTGGAAG 60
   AAAGTGAGG 69
(2) INFORMATION FOR SEQ ID NO:11:
   (i) SEQUENCE CHARACTERISTICS
      (A) LENGTH: 54 BASE PAIRS
      (B) TYPE: NUCLEIC ACID
      (C) STRANDEDNESS: SINGLE
      (D) TOPOLOGY: LINEAR
   (ii) MOLECULE TYPE: Oligonucleotide
   (xi) SEQUENCE DESCRIPTION: SEQ ID NO:11:
   ATTAACCCTC ACTAAAGGGA GGCCATGTGG AAATGGATAC TGACACATTG TGCC 54
(2) INFORMATION FOR SEQ ID NO:12:
   (i) SEQUENCE CHARACTERISTICS
      (A) LENGTH: 35 BASE PAIRS
      (B) TYPE: NUCLEIC ACID
      (C) STRANDEDNESS: SINGLE
      (D) TOPOLOGY: LINEAR
   (ii) MOLECULE TYPE: Oligonucleotide
   (xi) SEQUENCE DESCRIPTION: SEQ ID NO:12:
   CCCAAGCTTC CACAAACGTT GCCTTCCTCT ATGAG 35